# EUROPEAN PATENT APPLICATION

(11) **EP 4 303 217 A1**
(43) Date of publication of application: **10.01.2024**
(21) Application number: 22762638.9
(22) Date of filing: 04.03.2022
(51) Int. Cl.: C07D 403/10, C07D 403/12, C07D 253/02, A61K 31/53, A61P 5/14, A61P 1/16, A61P 3/04

(54) **AROMATIC COMPOUND, PREPARATION METHOD THEREFOR, AND APPLICATION THEREOF**

(30) Priority: 04.03.2021 CN 202110264844
(71) Applicant: Fukang (Shanghai) Health Technology Co., Ltd., Shanghai 201318 (CN)
(72) Inventor: SHEN, Xiaokun, Shanghai 201318 (CN); LIU, Huixin, Shanghai 201318 (CN)
(74) Representative: Potter Clarkson
(86) International application number: PCT/CN2022/079388
(87) International publication number: WO 2022/184177

(57) **Abstract**

The present invention discloses an aromatic compound, a method of preparation thereof and applications thereof. The present invention provides a compound as shown in Formula I or a pharmaceutically acceptable salt thereof. The compounds of the present invention have demonstrated good safety, tolerability and reduction of liver fat in mice and potential efficacy in the treatment of NASH in animal experiments in NASH disease models.

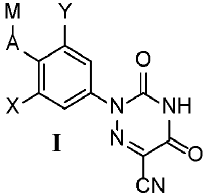

## Description

### Technical field

The invention belongs to the technical field of chemical medicine, and specifically relates to an aromatic compound, preparation method and application thereof.

### Background

The thyroid is a butterfly-shaped organ located at the base of the neck. The thyroid release controls the hormone of human body base metabolism namely thyroid hormone, so as to control the way the body uses energy. The thyroid hormone adjusts important bodily functions: breathing, heart rate, central and peripheral nervous system, weight, muscle strength, menstrual cycle, body temperature, cholesterol level and so on.

Thyroid hormone receptor (THR) receptor belongs to nuclear receptor superfamily which can be induced and expressed by thyroid hormone T3. The main subtype THR α -1 of thyroid hormone receptor, THR β-1 and THR β-2 are responsible for mediating thyroid hormone function, which is important to human growth and development and metabolism. THR β-1 is widely expressed in all tissues, but it is more prominent in brain, thyroid, liver and kidney, and THR β-2 is mainly in the anterior lobe of the brain, hypothalamus, retina, brain and inner ear in development expressed in tissue-specific manner.

The physiological effects of thyroid hormones are almost impacted on each organ system. Clinically, these effects are the change of lipid metabolism and the effect of cardiovascular development. Thyroid hormone has the beneficial effects of reducing cholesterol, improving blood fat characteristic and treating obesity. The thyroid hormone analog can reduce low density lipoprotein (LDL) - cholesterol, increase high density lipoprotein (HDL) cholesterol, stimulate reverse cholesterol transport and reduce plasma triglycerides and so on, and improve lipid profile.

Since THR β-1 is the same type of the main thyroid hormone receptor in the liver, if the normal activity of THR β-1 is inhibited by its mutant, it can be speculated that it causes metabolism. In addition, thyroid hormone also regulates apolipoprotein B. Apolipoprotein B is a major protein component of very low density lipoprotein (VLDL). Some research results show that, in addition to stimulating a lipid oxidation pathway, thyroid hormone inhibits lipid droplets as a lipid storage pathway, and promotes lipid droplets as part of the liposome of VLDL secretion.

The study demonstrated that a significantly higher proportion of patients with low thyroid function had nonalcoholic steatohepatitis (NASH) and advanced fibrosis compared with patients with completely normal thyroid function (NASH, 52.4% vs. 37.2%; advanced fibrosis, 21.0% vs. 10.6%; P < 0.01). In addition, patients with subclinical hypothyroidism were significantly more likely to have NASH and advanced fibrosis compared with patients with low levels of thyroid function (NASH, 57.6% vs. 48.8%; advanced fibrosis, 25.4% vs. 17.9%; P < 0.01). The serum thyroid-stimulating hormone levels were significantly higher in patients with NASH than in the normal group. In addition, thyroid function tests confirmed the presence of several thyroid abnormalities in patients with chronic liver disease. Furthermore, hypothyroidism was not associated with NASH and related to it with other known metabolic risk factors, i.e., hypothyroidism is an independent risk factor for NASH.

If the deleterious effects of thyroid hormone excess can be separated from the potential beneficial effects on cholesterol and lipid lowering, there is hope for the development of novel drugs with potent effects. Considering the roughly three-stage progression of non-alcoholic fatty liver disease (NAFLD) and NASH: i.e., fat deposition, hepatitis-hepatocyte death/apoptosis, and fibrosis/cirrhosis, the therapeutic strategy should include at least three aspects: i.e., reduction or elimination of hepatic fat deposition; control and suppression of persistent hepatic inflammation in the liver region/reducing hepatocyte death; and stopping the progression of fibrosis or degrading the fibre/extracellular matrix that has formed to reverse the fibrotic process. extracellular matrix to reverse the fibrotic process. Of these three aspects, reducing or eliminating fatty deposits and controlling and suppressing ongoing inflammation/reducing hepatocyte death are the most important. NAFLD / NASH is fundamentally a metabolic syndrome, which is closely related to disorders of fat metabolism, insulin resistance/type 2 diabetes, etc. At the same time, these metabolic disorders and fatty deposits and type 2 diabetes are also closely related. At the same time, these metabolic disorders and fat deposition lead to inflammation; inflammation leads to hepatocyte death/apoptosis; and hepatocyte death/apoptosis naturally progresses to fibrosis/cirrhosis. Most of the drugs currently in phase III clinical trials have the pharmacological mechanism of action of reducing or eliminating fat deposition/degeneration, controlling and suppressing persistent inflammation/reducing hepatocyte death.

In summary, the oral small molecule agonist for selective development of THR-beta target development is a very promising strategy, and there are already two pioneers in this respect. Among them, the candidate drug MGL-3196 of the United States Madrigal Pharmaceuticals Corporation has shown positive results in Phase II clinical trials conducted in patients with biopsy confirmed non-alcoholic steatohepatitis (NASH). MGL-3196 is a selective agonist of the liver-specific thyroid hormone receptor subtype (THR- β) that is orally administered once a day. In clinical research, using MRI-PDFF (a non-invasive imaging test) of liver fat percentage change as the main clinical end point, which displayed a statistically significant positive result, liver fat is reduced by more than 30 %, and there is a high correlation between the improvement of NASH on liver biopsy. In patients treated with MGL-3196, ALT and AST were observed to have a statistically significant decrease in ALT and AST, and additional secondary endpoints, such as LDL-C, triglycerides, apolipoprotein B and lipoprotein a, also showed a statistically significant improvement. These clinical indicators are associated with the clinical condition of the NASH patient. The medicine has entered the clinical phase III study in 2019.

In addition, Viking Therapeutics also developed an oral small molecule agonist VK2809 (or MB07811) with a thyroid hormone receptor beta subtype (THR- β) selectivity. The medicine is currently being researched in clinical phase II trials in primary hypercholesterolemia and non-alcoholic fatty liver disease patients. The results showed that the LDL-C levels in patients treated with VK2809 were significantly reduced by 20 % or more. Treatment of 12 weeks can significantly reduce the LDL-C level of NAFLD patients, and improve the liver fat content.

Because MGL-3196 and VK2809 reduce the effect of fat deposition/denaturation, the industry predicts that the thyroid hormone receptor beta subtype (THR- β) agonist has potential for treating NAFLD/NASH and metabolism syndrome. 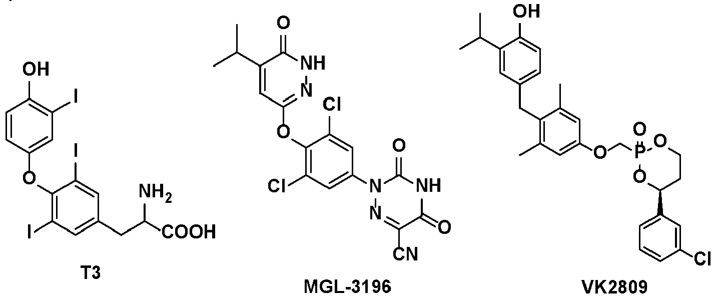 However, the currently reported THR-β agonists still have limitations, and the most important thing is to improve the agonistic activity and subtype selectivity of the compounds, especially for the THR-α subtype. The compounds currently under investigation still suffer from deficiencies in agonistic activity and selectivity. Only by breaking through the above bottlenecks, such compounds are expected to become breakthrough new therapies for NASH and related liver diseases.

### Contents of the Invention

The technical problem to be solved by the present invention is that existing agonists of thyroid hormone receptor THR-β are singly selective. To this end, the present invention provides an aromatic compound, its preparation method and its application. These agonists have significantly stronger agonistic activity on THR-β than the currently investigated drug MGL-3196, and the selection of THR-α subtypes is also significantly higher than that of MGL-3196. In animal experiments in NASH disease models, certain compounds have shown good safety, tolerability, and lowering of liver fat and hepatoprotective effects in mice.

The invention claims a compound represented by formula I or a pharmaceutically acceptable salt thereof, wherein the structure is as follows: 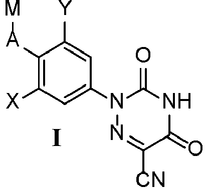
wherein A is O or CH₂;;
M is 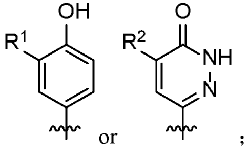
X and Y are independently chlorine, bromine, iodine, isotope ¹²⁴I or ¹³¹I of I or C₁ to C₆ alkyl;
R¹ is hydrogen, C₁ - C₆ alkyl, C₂ - C₆ alkenyl, one or more fluorine-substituted C₁ - C₆ alkyl, one or more fluorine-substituted C₂ - C₆ alkenyl, one or more deuterium-substituted C₁ - C₆ alkyl or one or more deuterium-substituted C₂ - C₆ alkenyl;
R² is C₂ - C₆ alkenyl, one or more fluorine-substituted C₁ - C₆ alkyl, one or more fluorine-substituted C₂ - C₆ alkenyl, one or more deuterium-substituted C₁ - C₆ alkyl or one or more deuterium-substituted C₂ - C₆ alkenyl.

In a certain scheme, the compound represented by formula I or a pharmaceutically acceptable salt thereof may further have the following definitions, the definition of the substituents relates to the definition of any one of the solutions of the invention (hereinafter referred to as "in a certain scheme"):
wherein A is O or CH₂;
X and Y are independently chlorine, bromine, iodine or C₁ to C₆ alkyl;
M is 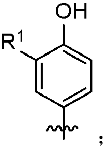 R¹ is hydrogen, C₁ - C₆ alkyl, C₂ - C₆ alkenyl, one or more fluorine-substituted C₁ - C₆ alkyl, one or more fluorine-substituted C₂ - C₆ alkenyl, one or more deuterium-substituted C₁ - C₆ alkyl or one or more deuterium-substituted C₂ - C₆ alkenyl;
or, M is 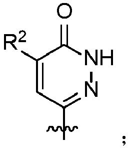 R² is one or more fluorine-substituted C₁ to C₆ alkyl.

In a certain scheme, wherein A is O, X and Y are independently chlorine, bromine or iodine;
M is 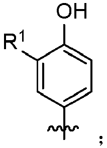 R¹ is C₂ to C₆ alkenyl or " one or more fluorine substituted C₁ to C₆ alkyl ".

In a certain scheme, wherein A is O, X and Y are independently chlorine or bromine; M is 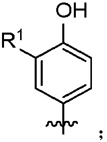 R¹ is C₂ - C₆ alkenyl or C₁ - C₆ alkyl substituted by one fluorine.

In a certain scheme, X and Y are independently chlorine, bromine, iodine or CH₃.

In a certain scheme, R¹ is C₂ - C₆ alkenyl or one or more fluorine-substituted C₁ - C₆ alkyl.

In a certain scheme, R² is a one or more fluorine-substituted C₁ to C₆ alkyl.

In a certain scheme, when R¹ is C₂ to C₆ alkenyl, the C₂ to C₆ alkenyl is C₂ to C₄ alkenyl. For example, R¹ is 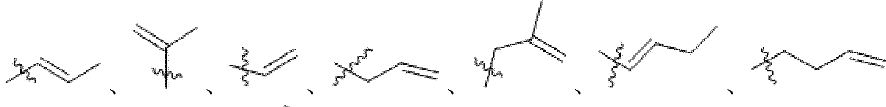 or 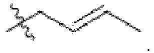 For example, R¹ is

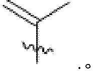

In a certain scheme, when R¹ is C₁ - C₆ alkyl, the C₁ - C₆ alkyl is C₁ - C₄ alkyl. For example R¹ is, methyl, ethyl, n-propyl, isopropyl, n-butyl, isobutyl, sec-butyl or tert-butyl; preferably isopropyl.

In a certain scheme, when R¹ is one or more fluorine-substituted C₁ - C₆ alkyl, the C₁ - C₆ alkyl is C₁ - C₄ alkyl. For example R¹ is methyl, ethyl, n-propyl, isopropyl, n-butyl, isobutyl, sec-butyl or tert-butyl; and one or more of the " one or more " is 1 or 3, preferably R¹ is

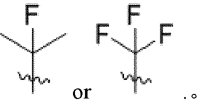

In a certain scheme, when R¹ is one or more deuterium-substituted C₁ - C₆ alkyl, the C₁ - C₆ alkyl is C₁ - C₄ alkyl. For example, R¹ is methyl, ethyl, n-propyl, isopropyl, n-butyl, isobutyl, sec-butyl or tert-butyl; and one or more of the " one or more " is 1 or 3, preferably R¹ is

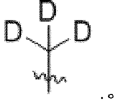

In a certain scheme, when R¹ is one or more fluorine-substituted C₂ to C₆ alkenyl, the C₂ to C₆ alkenyl is C₂ to C₄ alkenyl. For example, 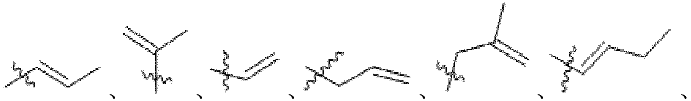 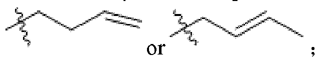 and one or more of the " one or more " is 1 or 3.

In a certain scheme, when R¹ is one or more deuterium-substituted C₂ to C₆ alkenyl, the C₂ to C₆ alkenyl is C₂ to C₄ alkenyl. For example, 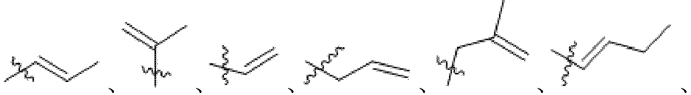 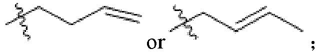 and one or more of the " one or more " is 1 or 3.

In a certain scheme, when R² is C₂ to C₆ alkenyl, the C₂ to C₆ alkenyl is C₂ to C₄ alkenyl. For example 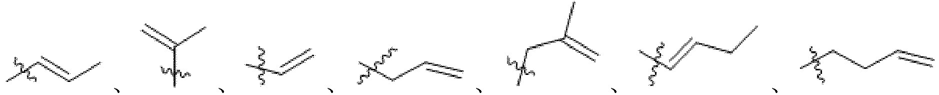 or 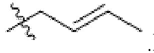 For example,

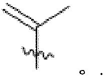

In a certain scheme, when R² is one or more fluorine-substituted C₁ - C₆ alkyl, the C₁ - C₆ alkyl is C₁ - C₄ alkyl. For example, methyl, ethyl, n-propyl, isopropyl, n-butyl, isobutyl, sec-butyl or tert-butyl; one or more of the " one or more " is 1 or 3, preferably R² is

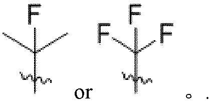

In a certain scheme, when R² is one or more deuterium-substituted C₁ - C₆ alkyl, the C₁ - C₆ alkyl is C₁ - C₄ alkyl. For example, methyl, ethyl, n-propyl, isopropyl, n-butyl, isobutyl, sec-butyl or tert-butyl; one or more of the " one or more " is 1 or 3, preferably R² is

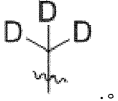

In a certain scheme, when R² is one or more fluorine-substituted C₂ to C₆ alkenyl, the C₂ to C₆ alkenyl is C₂ to C₄ alkenyl. For example, 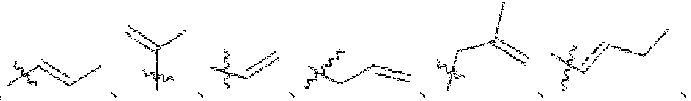 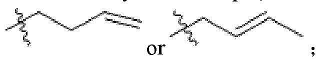 and one or more of the " one or more " is 1 or 3.

In a certain scheme, when R² is one or more deuterium substituted C₂ to C₆ alkenyl, the C₂ to C₆ alkenyl is C₂ to C₄ alkenyl. For example, 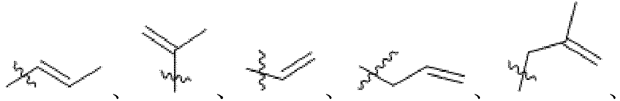 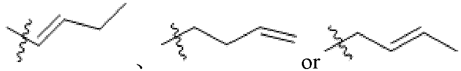 and one or more of the " one or more " is 1 or 3.

In a certain scheme, when X is C₁ - C₆ alkyl, the C₁ - C₆ alkyl is C₁ - C₄ alkyl. For example, methyl, ethyl, n-propyl, isopropyl, n-butyl, isobutyl, sec-butyl or tert-butyl; preferably methyl.

In a certain scheme, when Y is C₁ ~ C₆ alkyl, the C₁ ~ C₆ alkyl is C₁ ~ C₄ alkyl. For example, methyl, ethyl, n-propyl, isopropyl, n-butyl, isobutyl, sec-butyl or tert-butyl; preferably methyl.

In a certain scheme, R¹ is

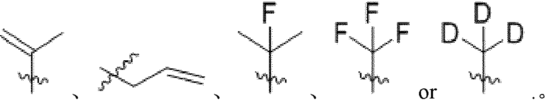

In a certain scheme,

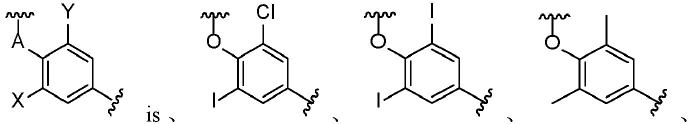

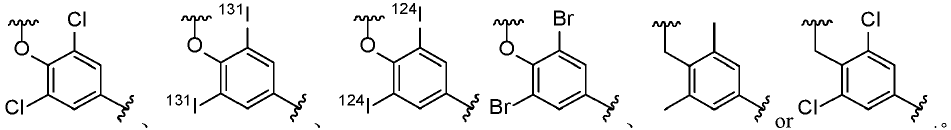

In a certain scheme, the compound represented by formula I is any one of the following compounds:

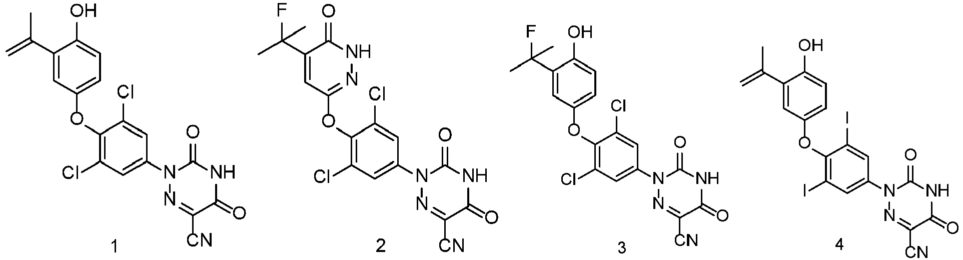

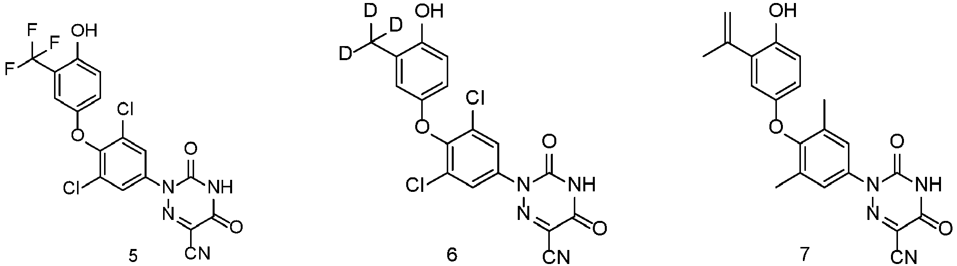

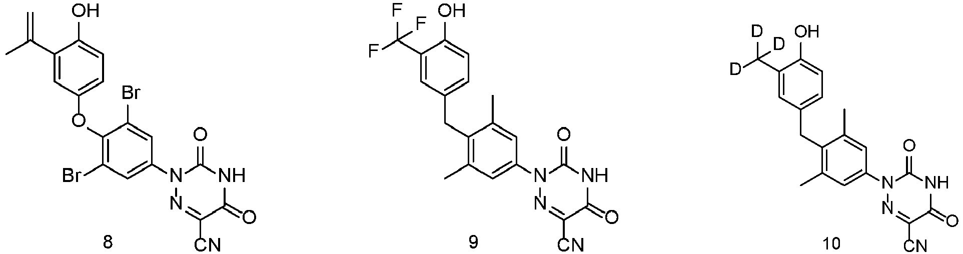

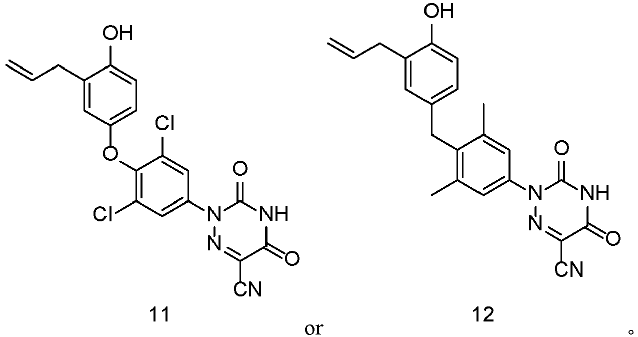

In the present invention, the compound represented by formula I or a pharmaceutically acceptable salt thereof, can be synthesized by a method similar to the known method in the chemical field, the steps and conditions can refer to the steps and conditions of a similar reaction in the field, especially according to the description of the synthesis. The starting materials are typically from commercial sources, or may be readily prepared using methods known to those skilled in the art (obtained by SciFinder, Reaxys online database).

The invention further claims a preparation method of the compound represented by formula I, comprising the following steps: in the solvent, and in the presence of alkali, the compound represented by formula II-a is subjected to the following ring-closing reaction, to obtain the compound represented by formula I; wherein the definition of M, X, Y and A is as said, R⁶ is C₁-C₆ alkyl;

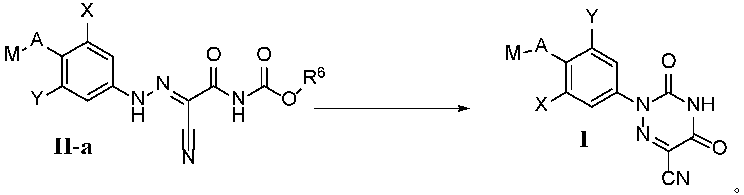

In a certain scheme, when R⁶ is C₁-C₆ alkyl, the C₁ to C₆ alkyl is C₁ to C₄ alkyl. For example, methyl, ethyl, n-propyl, isopropyl, n-butyl, primary butyl, isobutyl, sec-butyl or tert-butyl. For example, ethyl.

The invention further claims a compound represented by formula II-a, 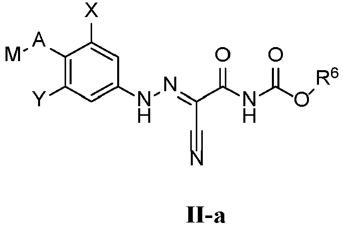 wherein M, A, X, Y and R⁶ are as defined above.

In a certain scheme, the compound represented by formula II-a is any one of the following compounds: 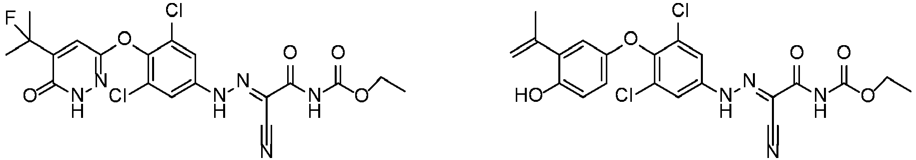 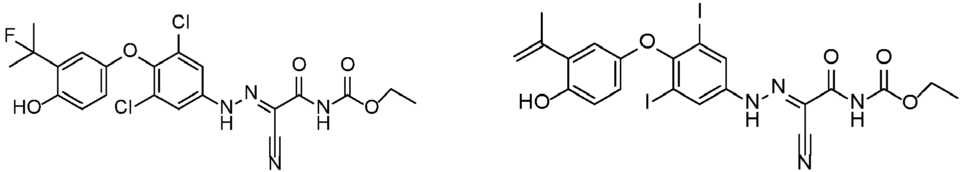 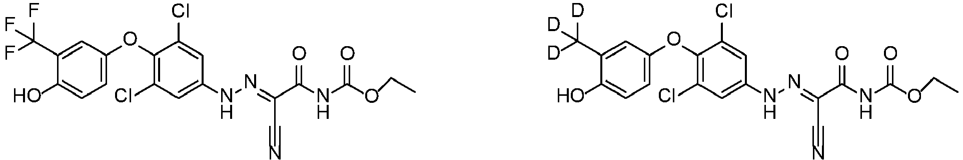 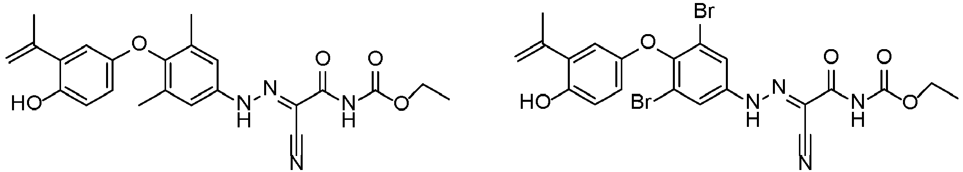 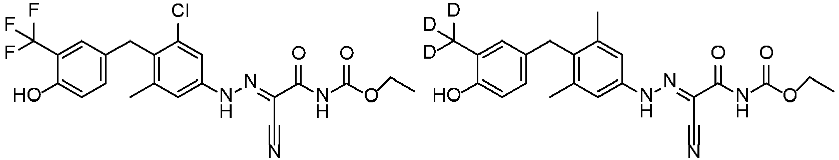 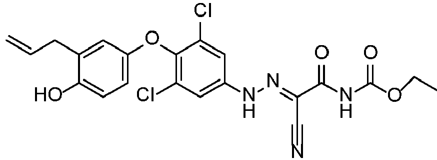 or

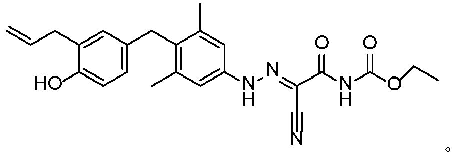

The present invention also provides a method for the preparation of a compound as shown in Formula II-a, which comprises the steps of: in a solvent, and in the presence of an acid, reacting a compound as shown in Formula II-b with sodium nitrite by diazotisation, and then reacting it with cyanoacetylcarbamic acid by the reaction shown in the following scheme, to obtain a compound as shown in Formula II-a; wherein M, A, X, Y, and R⁶ are defined as previously described;

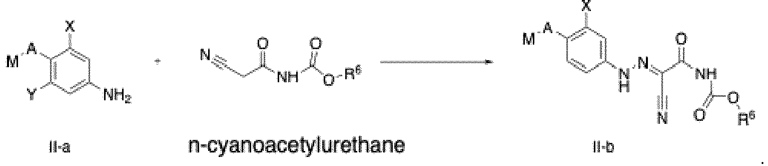

The invention claims a pharmaceutical composition, comprising a substance A and one or more pharmaceutically acceptable carriers. The substance A is the compound represented by formula I or a pharmaceutically acceptable salt thereof as hereinbefore described. In the pharmaceutical composition, the dosage of the compound represented by formula I or a pharmaceutically acceptable salt thereof can be a therapeutically effective amount.

The pharmaceutically acceptable carriers (pharmaceutical excipients) described may be those widely used in the field of drug production. Excipients are primarily used to provide a safe, stable and functional pharmaceutical composition, and may also provide means to dissolve the active ingredient at a desired rate after the subject has received administration, or to facilitate efficient absorption of the active ingredient after the subject has received administration of the composition. Said pharmaceutical excipients may be inert fillers or provide some function, such as stabilising the overall pH of the composition or preventing degradation of the active ingredient of the composition. Said pharmaceutical excipients may include one or more of the following excipients: binders, suspending aids, emulsifiers, diluents, fillers, granulators, adhesives, disintegrants, lubricants, anti-adhesive agents, flow aids, wetting agents, gelling agents, absorption delaying agents, solubility inhibitors, reinforcers, adsorbents, buffers, chelating agents, preservatives, colouring agents, flavour correcting agents, and sweeteners.

The pharmaceutical compositions of the present invention can be prepared using any method known to those of skill in the art based on the present disclosure. For example, conventional mixing, dissolving, granulating, emulsifying, grinding, encapsulating, embedding or freeze-drying process.

The invention further claims an application of substance B in preparing an agonist agent of THR-β.The substance B is the compound represented by formula I or a pharmaceutically acceptable salt thereof, or a pharmaceutical composition as described above.

In the application, the agonist agent of THR- β can be used in a mammal organism, it also can be used as an organism mainly as an experimental use, for example: as a standard sample or providing comparison to the sample, or according to the conventional method of the field to prepare the kit, providing fast detection for the agonist effect of THR- β.

The present invention also provides an application of a substance B in the preparation of a drug for use in the treatment and/or prevention of a disease associated with THR- β, said substance B being a compound as shown in formula I or a pharmaceutically acceptable salt thereof as described above, or a pharmaceutical composition as described above.

The disease is one or more of non-alcoholic fatty liver disease, obesity, liver fibrosis, 2 type diabetes and primary hypercholesterolemia.

Unless otherwise specified, the following terms present in the specification and claims of the present invention have the following meanings:
The term "pharmaceutically acceptable" refers to a salt, a solvent, an adjuvant and the like, generally non-toxic, safe, and suitable for use in a patient. The "patient" preferably is a mammal, more preferably a human.

The term "pharmaceutically acceptable salt" refers to a salt prepared by preparing a compound of the present invention with a relatively non-toxic, pharmaceutically acceptable acid or base. When the compound of the present invention contains a relatively acidic functional group, the alkali addition salt can be obtained by using a sufficient amount of a pharmaceutically acceptable base in a suitable inert solvent and an original contact of such a compound. Pharmaceutically acceptable alkali addition salts include, but are not limited to: lithium salt, sodium salt, potassium salt, calcium salt, aluminium salt, magnesium salt, zinc salt, bismuth salt, ammonium salt, diethanolamine salt. When the compound of the present invention contains a relatively basic functional group, the acid addition salt can obtained by using a sufficient amount of a pharmaceutically acceptable acid in a suitable inert solvent to react with such a compound. The pharmaceutically acceptable acid comprises inorganic acid, the inorganic acid comprises but is not limited to: hydrochloric acid, hydrobromic acid, hydroiodic acid, nitric acid, carbonic acid, phosphoric acid, phosphorous acid, sulphuric acid and so on. The pharmaceutically acceptable acid comprises organic acid, the organic acid comprises but is not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzene sulfonic acid, p-toluenesulfonic acid, citric acid, salicylic acid, tartaric acid, methanesulfonic acid, isonicotinic acid, oleic acid, tannic acid, pantothenic acid, tartaric acid hydrogen, ascorbic acid, gentisic acid, fumaric acid, gluconic acid, sugar acid, formic acid, ethanesulfonic acid, bis (4, 4'-methylene-bis (3-hydroxy-2-naphthoic acid)), amino acid (such as glutamic acid, arginine) and so on. When the compound of the present invention contains a relatively acidic and relatively basic functional group, it can be converted into a base addition salt or an acid addition salt. See Berge et al., "Pharmaceutical Salts", Journal of PharmaceuticalScience 66: 1-19 (1977), or, Handbook of Pharmaceutical Salts: Properties, Selection, and Use (P. Heinrich Stahl and Camille G. Werth, ed., Wiley-VCH, 2002).

When any variable (e.g., R¹) occurs several times in the definition of the compound, the definition of each position of the variable is independent of the definition of the rest of the occurrences of the position of said variable, their meanings are independent from each other, and do not affect each other. Therefore, if a certain group is substituted by 1, 2 or 3 R¹ groups, that is, the group may be substituted by at most 3 R¹, the definition of one of the positions of R¹ and the definition of the remaining positions of R¹ are independent from each other. In addition, combinations of the substituent and/or variables are allowed only when the combination produces a stable compound.

The total number of carbon atoms present in the group is represented by simplified symbols in front of certain chemical groups as defined herein. For example, C₁-C₆ alkyl refers to a total of 1, 2, 3, 4, 5, or 6 carbon atoms as defined in the above alkyl. The total number of carbon atoms in the simplified symbols does not include carbon that may be present in the substituent of the groups.

The term "treatment" refers to therapeutic therapy. When referring to specific conditions, the treatment refers to: (1) relieving one or more biological manifestations of a disease or disorder, (2) interference at (a) one or more points or (b) one or more biological manifestations in the biological cascade of the disease, (3) improving one or more symptoms associated with the disorder, influence or side effect, or one or more symptoms associated with the condition or a treatment, influence, or side effect thereof, or (4) one or more biological performance development of slowing the disease or disorder.

The term "therapeutically effective amount" refers to the amount of a compound sufficient to effectively treat the disease or condition described herein when administered to a patient. The "therapeutically effective amount" will vary depending upon the compound, the condition and the severity of the compound, and the age of the patient to be treated, and may be adjusted by one skilled in the art in accordance with the need.

It will be understood by those skilled in the art, according to the convention used in the field, the application of the formula of the description 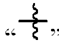 ' used in a group means that the corresponding group is connected with other fragments in the compound through the site.

The above preferred conditions can be combined randomly without departing from the common sense in the field, and each preferred embodiment of the present invention is obtained.

The reagent and raw material used in the invention can be sold in market.

The positive progress of the present invention is that:
The invention claims a thyroid hormone receptor agonist, preparation method and application thereof. The agonist activity of the agonist to THR-β is significantly stronger than the current clinical drug MGL-3196, and the selection of the THR-α subtype is also significantly higher than MGL-3196. The animal experiment of NASH disease model exhibits good safety, tolerance and the reduction effect of mouse liver fat and potential curative effect for treating NASH.

### Description of pictures

FIG. 1 is effect example 3 administering 42 days of animal weight change curve
FIG. 2 is the level of total cholesterol (TCHO) in the effect example 3;
FIG. 3 is an effect example 3 low density lipoprotein (LDL) level;
FIG. 4 is effect example 3 hepatocyte balloon-like degeneration score;
FIG. 5 is effect example 3 liver inflammation score;
FIG. 6 is effect example 3 liver fibrosis (Ishak) score.
FIG. 7 is effect example 3 liver NAS score.
FIG. 8 is the effect example 4 for 3 days animal weight change curve.

### Specific implementation examples

The present invention will be further described by way of example, but this is not intended to limit the present invention to the scope of the embodiments described. In the following examples, the experimental methods of specific conditions are not specified, the compounds are made according to the conventional methods and conditions, or according to the specification of the article.

### Example 1: 2 - (3, 5-dichloro-4 - (4-hydroxy-3 - (isopropenyl) phenoxy) phenyl) -3, 5-dioxo-2, 3, 4, 5-tetrahydro-1, 2, 4-triazine -6 - nitrile (compound 1)

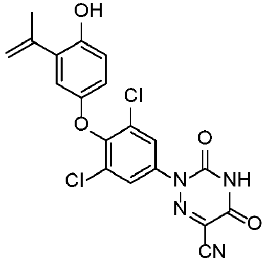

### Step 1: 2-isopropenyl benzene -1, 4-diol

To a solution of 2-bromobenzene-1,4-diol (5.0g, 226.5mmol) in 1, 4-dioxane (100 mL), potassium carbonate aqueous solution(11.0g, 79.4mol, 20 mL), isopropenylboronic acid pinacol ester (6.67g, 39.7mmol) and Pd (dppf) Cl₂ (0.97g, 1.32mmol) was added under nitrogen atmosphere. The mixture was heated to 105 °C. After stirring at 105°C for 5 hours, the reaction mixture was cooled down to room temperature and concentrated under reduced pressure to remove 1, 4-dioxane. Water (50 ml) and ethyl acetate(50 ml) was added into the mixture and the pH was adjusted to 2-3 with 10% aqueous HCl. The mixture was extracted with ethyl acetate (30 mL × 3) and the organic phase was combined. The organic phase was washed with saturated aqueous sodium chloride solution (10 ml). The organic phase was dried with anhydrous magnesium sulfate and filtered. The filtrate was concentrated and purified by silica gel column chromatography (eluent: petroleum : ethyl acetate = 10 : 1-5 : 1) to obtain 2.5 g of the target product in 63 % yield. ¹ H NMR (400MHz, CDCl₃): δ 6.80 (d, J=9.2Hz, 1H), 6.66-6.64 (m, 2H), 5.39 (t, J=1.6Hz, 1H), 5.30 (s, 1H), 5.14 (s, 1H), 4. 50 (s, 1H), 2.09 (s, 3H).

### Step 2: 4 - (2, 6-dichloro-4-nitrophenoxy) - 2 - (isopropenyl) phenol

To a solution of 2-isopropenylbenzene-1,4-diol (3.00 g, 20mmol) in acetonitrile (50 ml), sodium carbonate (7.57 g, 71.4 mmol), and 1,3-dichloro-2-fluoro-5-nitrobenzene (3 g, 14.3 mmol) were added. The mixture was stirred at 48 °C for 8 hours. After the reaction was complete, the reaction mixture was cooled down to room temperature and concentrated under reduced pressure to remove the acetonitrile. Water (50 ml) and ethyl acetate (50 ml) were added into the solution and the pH was adjusted to 2-3 with 10% aqueous HCl. The mixture was extracted with ethyl acetate (30 mL × 3) and the organic phase was combined. The organic phase was washed with saturated aqueous sodium chloride solution (10 ml). The organic phase was dried with anhydrous magnesium sulfate and filtered. The filtrate was concentrated and purified by silica gel column chromatography (eluent: petroleum : ethyl acetate = 20 : 1-15 : 1) to obtain 2.85 g of the target product in 59 % yield. ¹H NMR (400MHz, CDCl₃): δ 8.29 (s, 2H), 6.85 (d, *J*=8.8Hz, 1H), 6.67 (d, *J*=3.2Hz, 1H), 6.60 (dd, *J*=8.8, 3.2Hz, 1H), 5.46 (s, 1 H), 5.41 (t, *J*=1.2Hz, 1H), 5.17 (s, 1H), 2.08 (s, 3H).

### Step 3: 4 - (4-amino-2, 6-dichloro-phenoxy) - 2 - (isopropenyl) phenol

To a solution of 4 - (2, 6-dichloro-4-nitrophenoxy) - 2 - (isopropenyl) phenol (2.85g, 8.38mmol) in tetrahydrofuran (20 mL) and anhydrous methanol (20 mL) aqueous ammonium chloride (4.48g, 83.8mmol)(20 mL) and iron powder (2.82g, 50.3mmol) were added in a reaction flask. The mixture was stirred at 70 °C for 3 hours. After the reaction was complete, the reaction mixture was cooled down to room temperature. Water (50 ml), saturated aqueous sodium bicarbonate solution (30 ml) and ethyl acetate (50 ml) were added into the solution, stirred for 10 minutes, and then filtered. The filtrate was extracted with ethyl acetate (30 mL × 3) and organic phase was combined. The organic phase was washed with saturated aqueous sodium chloride solution (10 ml), then dried over anhydrous magnesium sulfate and filtered. The filtrate was concentrated to dryness under reduced pressure to give 2.42 g of product in 93 % yield. [M + H] ⁺ 310.1.¹H NMR (400MHz, CDCl₃): 6 6.81 (d, *J*=8.8Hz, 1H), 6.68-6.67 (m, 3H), 6.59 (dd, *J*=8.8, 3.2Hz, 1H), 5.38 (t, *J*=1.2Hz, 1H), 5. 36 (s, 1H), 5.15 (s, 1H), 3.72 (br s, 2H), 2.08 (s, 3H).

### Step 4: (2-cyano-2 - (2 - (3, 5-dichloro-4 - (4-hydroxy-3-isopropenyl-phenoxy) phenyl) - hydrazino) acetyl) carbamic acid ethyl ester

To a solution of 6M aqueous hydrochloric acid (40 ml) in anhydrous ethanol (30 ml) was added 4 - (4-amino-2, 6-dichlorophenoxy) -2-(isopropenyl) phenol (2.40 g, 7.74mmol) . The mixture was cooled down to 5 °C and an aqueous solution of sodium nitrite (534 mg,7.74 mmol)(3 ml) was added dropwise to the mixture. After the reaction was complete, water (30 ml) was added into the solution. During filtering, the filter cake was drenched with water (10 ml × 3). The filter cake was dried under reduced pressure to give 2.7 g of product in 73 %yield. [M + H] ⁺: 477.2.

### Step 5: 2 - (3, 5-dichloro-4 - (4-hydroxy-3 - (isopropenyl) phenoxy) phenyl) -3, 5-dioxo-2, 3, 4, 5-tetrahydro-1, 2, 4-triazine-6-carbonitrile

To a solution of 2-cyano-2 - (2 - (3, 5-dichloro-4 - (4-hydroxy-3-isopropenyl phenoxy) phenyl) hydrazone) acetyl) ethyl carbamate (2.70 g, 5.66mmol) in acetic acid (30 mL) sodium acetate (928 mg, 11.3 mmol) was added. The mixture was stirred at 118 °C for 3 hours. After the reaction was complete, the reaction mixture was cooled down to 60 °C and concentrated to dryness under reduced pressure. The concentrate was purified by preparative liquid phase purification over a C18 column (mobile phase: acetonitrile: 0.1% aqueous ammonium bicarbonate = 20%-80%) to give 1.05 g of product in 22% yield. [M-H]⁻: 428.8.¹HNMR (400MHz, DMSO-*d*₆): δ 13.29 (br s, 1H), 9.34 (s, 1H), 7.78 (s, 2H), 6.77 (d, *J*=8.8Hz, 1H), 6.66 (d, *J*=3.2Hz, 1H), 6.54 (dd, J=8.8, 3.2Hz, 1H), 5.10 (s, 2 H), 2.05 (s, 3H).

### Example 2: 2 - (3, 5-dichloro-4 - ((5 - (2-fluoropropane-2-yl) - 6-oxo-1, 6-dihydro-pyridazin-3-yl) oxy) phenyl-3, 5-dioxo-2, 3, 4, 5-tetrahydro-1, 2, 4-triazine-6-carbonitrile (compound 2)

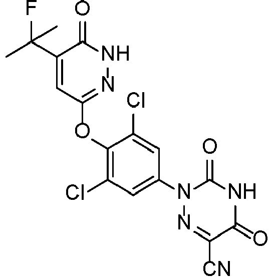

### step 1: 3, 6-dichloro - - 4 - (2-fluoro-propane-2-yl) - pyridazine

To a solution of 3,6-dichloropyridazine (17.6 g, 118 mmol) in concentrated sulfuric acid (17.4 g, 177 mmol) and water (110 mL) 2-fluoro-2-methylpropanoic acid (25 g, 236 mmol) was added. After stirring at 40 °C for 5 minutes, silver nitrate (2 g, 11.8 mmol) was added into the solution and the reaction was heated to 62 °C. A mixed solution of ammonium persulfate (45.8 g, 210 mmol) and water (220 mL) was added dropwise at 62 °C. After the reaction was stirred at 80 °C for an hour, the reaction solution was cooled down to 0-15 °C. Ammonium hydroxide was added dropwise into the solution to adjust the pH of the system to 9. The reaction was extracted with diisopropyl ether (200mL) and the organic phase was combined. The combined organic phase was washed with sodium bisulfite (100mL). The organic phase was dried with anhydrous sodium sulfate and filtered. The filtrate was concentrated and purified by silica gel column chromatography (eluent: petroleum : ethyl acetate = 10 : 1) to obtain 15.9 g of the target product in 64.9 % yield. MS (ESI) m/z: 209.0 [M + H] ⁺.

### Step 2: 3, 5-dichloro-4 - ((6-chloro-5 - (2-fluoropropane-2-yl) - pyridazin-3-yl) oxy) aniline

To a solution of 3,6-dichloro-4- (2-fluoropropan-2-yl)pyridazine (7.8 g, 37.5 mmol) in DMSO (78 mL) was added 4-amino-2,6-dichlorophenol (6.67 g, 27.5 mmol), potassium carbonate (20.7 g, 150 mmol), and CuI (4.27 g, 22.5 mmol). The reaction was stirred at 90 °Cfor 17h. After the reaction was completed, the reaction was cooled to room temperature. Water (500 ml) was added into the mixture. The mixture was extracted with ethyl acetate (30 mL × 3) and the organic phase was combined. The organic phase was washed with saturated aqueous sodium chloride solution (10 ml). The organic phase was dried with anhydrous magnesium sulfate and filtered. The filtrate was concentrated to obtain the crude 3,5-dichloro-4-((6-chloro-5-(2-fluoropropan-2-yl)pyridazin-3-yl)oxy)aniline (12.5 g). MS (ESI) m/z: 350.0 [M + H]⁺.

### Step 3: N - (3, 5-dichloro-4 - ((5 - (2-fluoropropane-2-yl) - 6-oxo-1, 6-dihydro-pyridazin-3-yl) oxy) phenyl) acetamide

To a solution of 3,5-Dichloro-4-((6-chloro-5-(2-fluoropropan-2-yl)pyridazin-3-yl)oxy)aniline (12.4 g, 35.8 mL) in glacial acetic acid, sodium acetate (10.3 g, 125 mmol) was added. The reaction was stirred at 100°C for 16 h. After the reaction was completed, the reaction solution was cooled down to room temperature and the pH of the system was adjusted to 8-9 with 1M sodium hydroxide aqueous solution. The mixture was extracted with ethyl acetate (100 mL ) and the organic phase was combined. The organic phase was washed with saturated aqueous sodium chloride solution (10 ml). The organic phase was dried with anhydrous magnesium sulfate and filtered. The filtrate was concentrated and purified by silica gel column chromatography (eluent: petroleum : ethyl acetate = 5 : 1-2 : 1) to obtain N-(3,5-dichloro-4-((5-(2-fluoropropan-2-yl)-6-oxo-1,6-dihydropyridazin-3-yl)oxy). -yl)oxy)phenyl)acetamide (2.28 g, 16.3% yield). MS (ESI) m/z: 373.1 [M + H]⁺.

### Step 4 6 - (4-amino-2, 6-dichloro-phenoxy) - 4 - (2-fluoro-propane-2-yl) - pyridazin-3 (2H) - one

To a solution of N-(3,5-dichloro-4-((5-(2-fluoropropan-2-yl)-6-oxo-1,6-dihydropyridazin-3-yl)oxy)phenyl)acetamide (1.0 g, 2.7 mmol) in ethanol (20 mL), 6N hydrochloric acid (24 mL) was added. The reaction mixture was stirred at 70 °C for 2.5 h. After the reaction was completed, the reaction solution was cooled to room temperature. Then the reaction was filtered, the filter cake was washed with water, and the solid was dried under reduced pressure to give 6-(4-amino-2,6-dichlorophenoxy)-4-(2-fluoropropan-2-yl)pyridazin-3(2H)-one (749 mg, yield: 84%). MS (ESI) m/z: 331.1 [M + H] ⁺.

### Step 5 (2-cyano-2 - (2 - (3, 5-dichloro-4 - ((5 - (2-fluoro-propyl-2-yl) - 6-oxo-1, 6-dihydro-pyridazin-3-yl) oxy) phenyl) - hydrazino) acetyl) carbamic acid ethyl ester

To a solution of 6-(4-amino-2,6-dichlorophenoxy)-4-(2-fluoropropan-2-yl)pyridazin-3(2H)-one (500 mg, 1.51 mmol) in 4N aqueous hydrochloric acid (22.5 mL), 0.26N aqueous sodium nitrite (7.5 mL) was added dropwise at 0°C. After the dropwise addition, the reaction was stirred at 0°C for 2h. Then the reaction mixture was filtered. The filtrate was added dropwise to a mixed solution of ethyl cyanoacetylcarbamate (236 mg, 1.51 mmol), pyridine (9.2 mL), and water (30 mL). The reaction was stirred at 0°C for 1.5 h. After the reaction was completed, the mixture was filtered. The filter cake was washed with water (10 mL) and petroleum ether (10 mL), and the solid was dried under reduced pressure to obtain (2-cyano-2-(2-(3-(3 ,5-dichloro-4-((5-(2-fhioropropyl-2-yl)-6-oxo-1,6-dihydropyridazin-3 -yl)oxy)phenyl)hydrazinylidene)acetyl)carbamic acid ethyl ester (218 mg, yield 29%). MS (ESI) m/z: 498.1 [M + H]⁺.

### step 62 - (3, 5-dichloro-4 - ((5 - (2-fluoropropane-2-yl) - 6-oxo-1, 6-dihydro-pyridazin-3-yl) oxy) phenyl -3, 5-dioxo-2, 3, 4, 5-tetrahydro-1, 2, 4-triazine-6-carbonitrile

To a solution of (2-cyano-2-(2-(3,5-dichloro-4-((5-(2-fluoropropan-2-yl)-6-oxo-1,6-dihydropyridazin-3-yl)oxy)phenyl)hydrazinyl)acetyl)carbamate (254 mg, 0.5 mmol) in glacial acetic acid (4.8 mL), sodium acetate (208 mg, 2.5 mmol) was added. The reaction mixture was stirred 120°C for 2 hours. Then the reaction solution was cooled down to 60°C and concentrated to dryness under reduced pressure. The concentrate was purified by preparative liquid phase purification on a C18 column (mobile phase: acetonitrile: aqueous solution = 20%-95%) to give 55 mg of product in 24.2% yield. MS (ESI) m/z: 452.0 [M + H]⁺. H NMR (400MHz, DMSO-*d*₆): δ 7.79 (s, 2H), 7.50 (s, 1H), 1.74 (s, 3H), 1.68 (s, 3H).

### Example 3 2 - (3, 5-dichloro-4 - (3 - (2-fluoro-propane-2-yl) - 4-hydroxy-phenoxy) - phenyl) - 3, 5-dioxo-2, 3, 4, 5-tetrahdro-1, 2, 4-triazine-6-carbonitrile (compound 3)

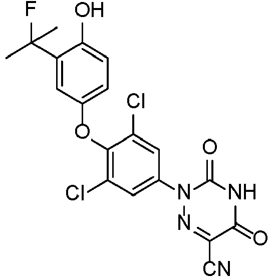

### step 1 4 - (2, 6-dichloro-4-nitrophenoxy) - 2 - (2-fluoropropane-2-yl) phenol

To a solution of 2-(2-fluoropropan-2-yl)benzene-1,4-diol (3.00 g, 17.6 mmol) in acetonitrile (50 mL) sodium carbonate (6.66 g, 63 mmol), and 1,3-dichloro-2-fluoro-5-nitrobenzene (2.65 g, 12.6 mmol) were added. The reaction was stirred at 50°C for 8 hours. After the reaction was complete, the reaction solution was cooled down to room temperature and concentrated under reduced pressure to remove the acetonitrile. Water (50 mL) and ethyl acetate (50 mL) were added into the solution, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrated was concentrated and purified by silica gel column chromatography(eluent: petroleum ether/ethyl acetate=20:1~15:1) to give 3.2 g, yield 70%. MS (ESI) m/z: 361.0 [M + H] ⁺

### step 2 4 - (4-amino-2, 6-dichloro-phenoxy) - 2 - (2-fluoro-propane-2-yl) phenol

To a solution of 4-(2,6-dichloro-4-nitrophenoxy)-2-(2-fluoropropan-2-yl)phenol (3.0 g, 8.38 mmol) in tetrahydrofuran (20 mL) and anhydrous methanol (20 mL), ammonium chloride (4.48 g, 83.8 mmol) aqueous solution (20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.35 g of product in 85% yield. MS (ESI) m/z: 331.1 [M + H] +.

### Step 3 (2-cyano-2 - (2 - (3, 5-dichloro-4 - (3 - (2-fluoropropane-2-yl) - 4-hydroxy-phenoxy) phenyl) - hydrazino) acetyl) carbamic acid ethyl ester

To a solution of 6M aqueous hydrochloric acid (40 mL) in anhydrous ethanol (30 mL), 4-(4-amino-2,6-dichlorophenoxy)-2-(2-fluoropropan-2-yl)phenol (2.30 g, 6.96 mmol) was added. The mixture was cooled down to 5°C and an aqueous solution of sodium nitrite (534 mg, 7.74 mmol) (3 mL) was added dropwise to the mixture. After dropwise addition, the mixture was continued to stir for 30 min at 0-5°C. A solution of pyridine (30 mL), water (40 mL) and n-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.55 g of product in 72% yield. MS (ESI) m/z: 497.4 [M + H]⁺.

### Step 4 2 - (3, 5-dichloro-4 - (3 - (2-fluoropropane-2-yl) - 4-hydroxy-phenoxy) - phenyl) - 3, 5-dioxo-2, 3, 4, 5-tetrahydro-1, 2, 4-triazine-6-carbonitrile

To a solution of ethyl (2-cyano-2-(2-(3,5-dichloro-4-(3-(2-fluoropropan-2-yl)-4-hydroxyphenoxy)phenyl)hydrazinyl)acetyl)carbamate (2.50 g, 5.03 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.02 g of product in 45% yield. MS (ESI) m/z: 452.3 [M + H]⁺. ¹ HNMR (400MHz, DMSO-*d*₆): δ 13.24 (br s, 1H), 7.79 (s, 2H), 7.08 (d, 1H), 7.05 (d, 1H), 6.83 (dd, 1H), 1.70 (d, 3H), 1.68 (d, 3H)

### Example 4 2 - (3, 5-Diiodo-4 - (4-hydroxy-3 - (isopropenyl) phenoxy) phenyl) -3, 5-dioxo-2, 3, 4, 5-tetrahydro-1, 2, 4-triazine-6-carbonitrile (compound 4)

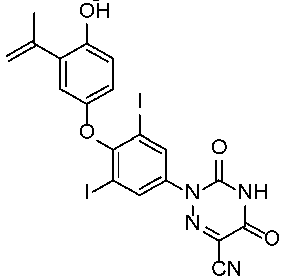

### step 1 4 - (2, 6-diiodo - -4 - nitrophenoxy) -2-(isopropenyl) phenol

To a solution of 2-isopropenylbenzene-1,4-diol (3.00 g, 20.0 mmol) in acetonitrile (50 mL), sodium carbonate (7.57 g, 71.4 mmol), and 1,3-diiodo-2-fluoro-5 nitrobenzene (5.62 g, 14.3 mmol) were added. The reaction was stirred at 48°C for 8 hours. After the reaction was complete, the reaction solution was cooled down to room temperature and concentrated under reduced pressure to remove the acetonitrile. To the concentrate, water (50 mL) and ethyl acetate (50 mL) were added, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrate was concentrated and purified by silica gel column chromatography (eluent: petroleum : ethyl acetate = 20 : 1-15 : 1) to obtain 4.86 g of the target product in 65 % yield. MS (ESI) m/z: 524.1 [M + H]⁺.

### step 2 4 - (4-amino-2, 6-diiodo-phenoxy) - 2 - (isopropenyl) phenol

To a solution of 4-(2,6-diiodo-4-nitrophenoxy)-2-(isopropenyl)phenol (4.38 g, 8.38 mmol) in tetrahydrofuran (20 mL) and anhydrous methanol (20 mL), ammonium chloride aqueous solution(4.48 g, 83.8 mmo, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 3.72 g of product in 90% yield.MS (ESI) m/z: 494.1 [M + H]⁺_{∘}

### Step 3 (ethyl 2-cyano-2-(2-(3,5-diiodo-4-(4-hydroxy-3-isopropenylphenoxy)phenyl)hydrazinylidene)acetyl)carbamate

To a solution of 6M aqueous hydrochloric acid (40 mL) in anhydrous ethanol (30 mL), 4-(4-amino-2,6-diiodophenoxy)-2-(isopropenyl)phenol (3.82 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture continued to be stirred for 30 min at 0-5°C. A solution of pyridine (30 mL), water (40 mL), and ethyl cyanoacetylcarbamate (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 3.47 g of product in 68% yield.MS (ESI) *m*/*z:* 661.2 [M + H]⁺_{∘}

### step 4 2 - (3, 5-diiodo-4 - (4-hydroxy-3 - (isopropenyl) phenoxy) phenyl) -3, 5-dioxo-2, 3, 4, 5-tetrahydro-1, 2, 4-triazine-6-carbonitrile

To a solution of ethyl (2-cyano-2-(2-(3,5-diiodo-4-(4-hydroxy-3-isopropenylphenoxy)phenyl)hydrazinylidene)acetyl)carbamic acid ethyl ester (3.74 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%-80%) to give 1.91 g of product in 55% yield. MS (ESI) *m*/*z:* 615.1 [M + H]⁺_{∘} ¹H NMR (400 MHz, DMSO-*d*₆): δ 13.10 (br s, 1H), 9.50 (s, 1H), 7.93 (s, 2H), 7.15 (m, 2H), 6.90 (s, 1H), 5.10 (d, 2H), 2.43 (s, 1H)_{∘}

### Example 5 2 - (3, 5-dichloro-4 - (4-hydroxy-3 - (trifluoromethyl) phenoxy) phenyl) -3, 5-dioxo-2, 3, 4, 5-tetrahdro-1, 2, 4-triazine-6-carbonitrile (compound 5)

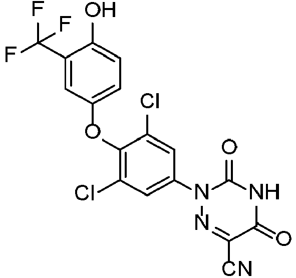

### step 1 4 - (2, 6-dichloro - -4 - nitrophenoxy) -2-(trifluoromethyl) phenol

To a solution of 2-trifluoromethylbenzene-1,4-diol (3.56 g, 20.0 mmol) in acetonitrile (50 mL), sodium carbonate (7.57 g, 71.4 mmol) and 1,3-dichloro-2-fluoro-5 nitrobenzene (3.00 g, 14.3 mmol) were added. The reaction was stirred at 48°C for 8 hours. After the reaction was complete, the reaction solution was cooled down to room temperature and concentrated under reduced pressure to remove the acetonitrile. Water (50 mL) and ethyl acetate (50 mL) were added to the concentrate, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrate was concentrated and purified by silica gel column chromatography (eluent: petroleum : ethyl acetate = 20 : 1-15 : 1) to obtain 3.32 g of the target product in 62 % yield. MS (ESI) m/z: 369.1 [M + H] ⁺.

### step 2 4 - (4-amino-2, 6-dichloro-phenoxy) - 2 - (trifluoromethyl) phenol

To a solution of 4-(2,6-dichloro-4-nitrophenoxy)-2-(trifluoromethyl)phenol (3.08 g, 8.38 mmol) in tetrahydrofuran (20 mL) and anhydrous methanol (20 mL), ammonium chloride aqueous solution(4.48 g, 83.8 mmo, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.55 g of product in 90% yield.MS (ESI) *m*/*z:* 339.1 [M + H]⁺_{∘}

### Step 3 (2-cyano-2 - (2 - (3, 5-dichloro-4 - (4-hydroxy-3-trifluoromethyl-phenoxy) phenyl) - hydrazino) acetyl) carbamic acid ethyl ester

To a solution of 6M aqueous hydrochloric acid (40 mL) in anhydrous ethanol (30 mL), 4-(4-amino-2,6-dichlorophenoxy)-2-(trifluoromethyl)phenol (2.62 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture continued to be stirred for 30 min at 0-5°C. A solution of pyridine (30 mL), water (40 mL), and ethyl (2-cyanoacetyl)carbamate (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.93 g of product in 75% yield. MS (ESI) *m*/*z:* 506.3 [M + H]⁺_{∘}

### Step 4 2 - (3, 5-dichloro-4 - (4-hydroxy-3 - (trifluoromethyl) phenoxy) phenyl) -3, 5-dioxo-2, 3, 4, 5-tetrahydro-1, 2, 4-triazine-6-carbonitrile

To a solution of (2-cyano-2-(2-(3,5-dichloro-4-(4-hydroxy-3-trifluoromethylphenoxy)phenyl)hydrazinylidene)acetyl)carbamic acid ethyl ester (2.70 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.3 g of product in 50% yield.MS (ESI) *m*/*z*: 460.1 [M + H]⁺.¹HNMR (400 MHz, DMSO-*d*₆): δ 13.0 (br s, 1H), 9.70 (s, 1H),7.80 (s, 2H), 7.38 (d, , 1H), 7.26 (d, 1H), 6.84 (d, 1H).

### Example 62 - (3, 5-Dichloro-4 - (4-hydroxy-3 - (deuterated methyl) phenoxy) phenyl) -3, 5-dioxo-2, 3, 4, 5-tetrahdro-1, 2, 4-triazin-6-carbonitrile (Compound 6)

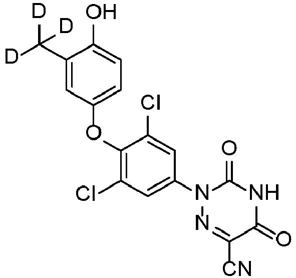

### Step 1 4 - (2, 6-dichloro-4-nitrophenoxy) - 2 - (deuterated methyl) phenol

To a solution of 2-deuteromethylbenzene-1,4-diol (2.55 g, 20.0 mmol) in acetonitrile (50 mL), sodium carbonate (7.57 g, 71.4 mmol), and 1,3-dichloro-2-fluoro-5 nitrobenzene (3.00 g, 14.3 mmol) were added. The reaction was stirred at 48°C for 8 hours. After the reaction was complete, the reaction solution was cooled down to room temperature and concentrated under reduced pressure to remove the acetonitrile. Water (50 mL) and ethyl acetate (50 mL) were added to the concentrate, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrate was concentrated and purified by silica gel column chromatography (eluent: petroleum : ethyl acetate = 20 : 1-15 : 1) to obtain 2.95 g of the target product in 65 % yield.MS (ESI) *m*/*z:* 318.1 [M + H]⁺_{∘}

### step 2 4 - (4-amino-2, 6-dichloro-phenoxy) - 2 - (deuterated methyl) phenol

To a solution of 4-(2,6-dichloro-4-nitrophenoxy)-2-(deuteromethyl)phenol (2.66 g, 8.38 mmol) in tetrahydrofuran (20 mL) and anhydrous methanol (20 mL), ammonium chloride aqueous solution(4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.24 g of product in 93% yield. MS (ESI) *m*/*z:* 288.2 [M + H]⁺.

### Step 3 (2-cyano-2 - (2 - (3, 5-dichloro-4 - (4-hydroxy-3-deuterated methyl phenoxy) phenyl) - hydrazino) acetyl) carbamic acid ethyl ester

To a solution of 6M aqueous hydrochloric acid (40 mL) in anhydrous ethanol (30 mL), 4-(4-amino-2,6-dichlorophenoxy)-2-(deuteromethyl)phenol (2.22 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture was continued to stir for 30 min at 0~5°C. A solution of pyridine (30 mL), water (40 mL), and ethyl (2-cyanoacetyl)carbamate (1.12 g, 7.74 mmol) was added dropwise to the reaction solution and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.6 g of product in 75% yield.MS (ESI) *m*/*z:* 455.3 [M + H]⁺.

### Step 4 2 - (3, 5-dichloro-4 - (4-hydroxy-3 - (deuterated methyl) phenoxy) phenyl) -3, 5-dioxo-2, 3, 4, 5-tetrahydro-1, 2, 4-triazine-6-carbonitrile

To a solution of (2-cyano-2-(2-(3,5-dichloro-4-(4-hydroxy-3-deuteromethylphenoxy)phenyl)hydrazinylidene)acetyl)carbamic acid ethyl ester (2.70 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.04 g of product in 45% yield.MS (ESI) m/z: 409.2 [M + H]+.¹H NMR (400 MHz, DMSO-*d*₆): δ 13.50 (br s, 1H), 9.41 (s, 1H), 7.84 (s, 2H), 7.07 (d, 1H), 6.99 (s, 1H), 6.79 (d, 1H).

### Example 7 2 - (3, 5-dimethyl-4 - (4-hydroxy-3 - (isopropenyl) phenoxy) phenyl) -3, 5-dioxo-2, 3, 4, 5-tetrahdro-1, 2, 4-triazin-6-carbonitrile (compound 7)

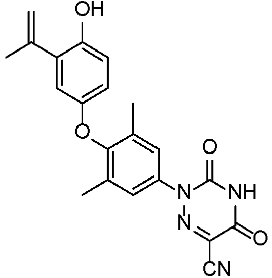

### step 1 4 - (2, 6-dimethyl - -4 - nitrophenoxy) -2-(isopropenyl) phenol

To a solution of 2-isopropenylbenzene-1,4-diol (3.00 g, 20.0 mmol) in acetonitrile (50 mL), sodium carbonate (7.57 g, 71.4 mmol), and 1,3-dimethyl-2-fluoro-5 nitrobenzene (2.42 g, 14.3 mmol) were added. The reaction was stirred at 48°C for 8 hours. After the reaction was complete, the reaction solution was cooled down to room temperature and concentrated under reduced pressure to remove the acetonitrile. Water (50 mL) and ethyl acetate (50 mL) were added to the concentrate, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrate was concentrated and purified by silica gel column chromatography (eluent: petroleum : ethyl acetate = 20 : 1-15 : 1) to obtain 2.99 g of the target product in 69.8 % yield.MS (ESI) m/z: 300.3 [M + H]⁺.

### step 2 4 - (4-amino-2, 6-dimethyl-phenoxy) - 2 - (isopropenyl) phenol

To a solution of 4-(2,6-dimethyl-4-nitrophenoxy)-2-(isopropenyl)phenol (2.85 g, 8.38 mmol) in tetrahydrofuran (20 mL) and anhydrous methanol (20 mL), ammonium chloride aqueous solution(4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.14 g of product in 95% yield.MS (ESI) m/z: 270.3 [M + H]⁺.

### Step 3 (2-cyano-2 - (2 - (3, 5-dimethyl-4 - (4-hydroxy-3-isopropenyl phenoxy) phenyl) - hydrazino) acetyl) carbamic acid ethyl ester

To a solution of 6M aqueous hydrochloric acid (40 mL) in anhydrous ethanol (30 mL) was added 4-(4-amino-2,6-dimethylphenoxy)-2-(isopropenyl)phenol (2.08 g, 7.74 mmol). The mixture was cooled down to 5°C and an aqueous solution of sodium nitrite (534 mg, 7.74 mmol) (3 mL) was added dropwise to the mixture. After dropwise addition, the mixture was continued to stir for 30 min at 0~5°C. A solution of pyridine (30 mL), water (40 mL), and n-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.53 g of product in 75% yield. MS (ESI) m/z: 437.5 [M + H]⁺_{∘}

### Step 4 2 - (3, 5-dimethyl-4 - (4-hydroxy-3 - (isopropenyl) phenoxy) phenyl) -3, 5-dioxo-2, 3, 4, 5-tetrahydro-1, 2, 4-triazine-6-carbonitrile

To a solution of (2-cyano-2-(2-(2-(3,5-dimethyl-4-(4-hydroxy-3-isopropenylphenoxy)phenyl)hydrazinylidene)acetyl)carbamic acid ethyl ester (2.47 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.05 g of product in 22% yield.MS (ESI) m/z: 391.4 [M + H]⁺_{∘} ¹H NMR (400 MHz, DMSO-d6): δ 13.40 (br s, 1H), 9.38 (s, 1H), 7.50 (s, 2H), 7.16 (m, 2H), 6.91 (s, 1H), 5.15 (s, 2H), 2.30 (s, 3H), 2.15 (s, 6H)_{∘}

### Example 8 2 - (3, 5-dibromo-4 - (4-hydroxy-3 - (isopropenyl) phenoxy) phenyl) -3, 5-dioxo-2, 3, 4, 5-tetrahydro-1, 2, 4-triazine-6-carbonitrile (compound 8)

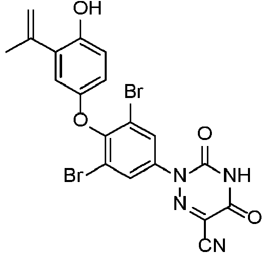

### step 1 4 - (2, 6 - -4 - nitrophenoxy) -2-(isopropenyl) phenol

To a solution of 2-isopropenylbenzene-1,4-diol (3.00 g, 20.0 mmol) in acetonitrile (50 mL), sodium carbonate (7.57 g, 71.4 mmol) and 1 1,3-dibromo-2-fluoro-5 nitrobenzene (4.28 g, 14.3 mmol) were added. The reaction was stirred at 48°C for 8 hours. After the reaction was complete, the reaction solution was cooled down to room temperature and concentrated under reduced pressure to remove the acetonitrile. Water (50 mL) and ethyl acetate (50 mL) were added to the concentrate, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrate was concentrated and purified by silica gel column chromatography (eluent: petroleum : ethyl acetate = 20 : 1-15 : 1) to obtain 3.68 g of the target product in 60 % yield.MS (ESI) m/z: 430.1 [M + H]⁺_{∘}

### step 2 4 - (4-amino-2, 6-bromo-phenoxy) - 2 - (isopropenyl) phenol

To a solution of 4-(2,6-dibromo-4-nitrophenoxy)-2-(isopropenyl)phenol (3.59 g, 8.38 mmol) in tetrahydrofuran (20 mL) and anhydrous methanol (20 mL), ammonium chloride aqueous solution(4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 3.18 g of product in 95% yield. MS (ESI) m/z: 400.1 [M + H]⁺_{∘}

### Step 3 (2-cyano-2 - (2 - (3, 5-dibromo-4 - (4-hydroxy-3-isopropenyl phenoxy) phenyl) - hydrazino) acetyl) carbamic acid ethyl ester

To a solution of 6M aqueous hydrochloric acid (40 mL) in anhydrous ethanol (30 mL), 4-(4-amino-2,6-dibromophenoxy)-2-(isopropenyl)phenol (3.09 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture continued to be stirred for 30 min at 0-5°C. A solution of pyridine (30 mL), water (40 mL) and n-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 3.24 g of product in 74% yield.MS (ESI) m/z: 567.2 [M + H]⁺_{∘}

### step 4 2 - (3, 5-dibromo-4 - (4-hydroxy-3 - (isopropenyl) phenoxy) phenyl) -3, 5-dioxo-2, 3, 4, 5-tetrahydro-1, 2, 4-triazine-6-carbonitrile

To a solution of (2-cyano-2-(2-(3,5-dibromo-4-(4-hydroxy-3-isopropenylphenoxy)phenyl)hydrazinylidene)acetyl)carbamic acid ethyl ester (3.20 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.32 g of product in 45%yield.MS (ESI) m/z: 521.1 [M + H]⁺_{∘} 1H NMR (400 MHz, DMSO-d6): δ 13.35 (br s, 1H), 9.45 (s, 1H), 7.77 (s, 2H), 7.14 (m, 2H), 6.91 (s, 1H), 5.10 (s, 2H), 2.35 (s, 3H)_{∘}

### Example 9 2 - (4 - (4-hydroxy-3 - (trifluoromethyl) benzyl) -3, 5-dimethylphenyl) -3, 5-dioxo-2, 3, 4, 5-tetrahydro-1, 2, 4-triazin-6-carbonitrile (compound 9)

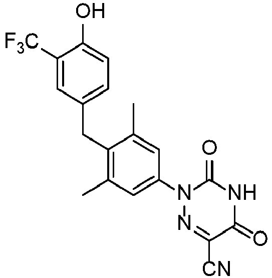

### step 1 (2, 6-dimethyl - -4 - nitrophenyl) (4-methoxy - -3-(trifluoromethyl) phenyl) methanol

To a solution of 2-bromo-1,3-dimethyl-5-nitrobenzene (2.83 g, 12.3 mmol) in tetrahydrofuran (80 mL), n-butyllithium (10 mL, 24.5 mmol) was added dropwise at -70° C.
After being stirred at -70° C for 20 min, 4-methoxy-3-(trifluoromethyl)benzaldehyde (3.0 g, 14.7 mmol) in tetrahydrofuran (20mL) was added dropwise into the above reaction solution, and the reaction was stirred at - 70° C for 60 min. After the reaction was completed, the reaction solution was poured into saturated aqueous ammonium chloride solution (300mL). The mixture was extracted with ethyl acetate (150mL × 2) and the organic phases were combined. The organic phase was washed with saturated sodium chloride (200 mL), then dried over anhydrous magnesium sulfate and filtered. The filtrate was concentrated and purified by reversed-phase preparative liquid phase purification (mobile phase A: water, mobile phase B: acetonitrile, gradient: 10-95% (%B)) to give 2.84 g of product in 65% yield. MS (ESI) m/z: 336.3 [M + H]⁺_{∘}

### step 2 2 - (4-methoxy - -3-(trifluoromethyl) benzyl) -1, 3-dimethyl-5-nitrobenzene

To a solution of (2,6-dimethyl-4-nitrophenyl)(4-methoxy-3-(trifluoromethyl)phenyl)methanol (2.75 g, 7.74 mmol) in dichloromethane (40 mL), trifluoroacetic acid (10 mL) and triethylsilane (20 mL) were added dropwise at room temperature. The reaction was stirred at room temperature overnight. After the reaction was completed, the reaction solution was poured into saturated aqueous sodium bicarbonate solution and extracted by dichloromethane (300 mL × 2).The organic phases were combined and washed with saturated aqueous sodium chloride solution (250 mL). The combined organic phase was dried with anhydrous magnesium sulfate, then filtered. The filtrate was concentrated and purified by reversed-phase preparative liquid phase purification (mobile phase A: water, mobile phase B: acetonitrile, gradient: 5-85% (%B)) to give 1.58 g of product in 60% yield. MS (ESI) m/z: 340.3 [M + H]⁺_{∘}

### step 3 4 - (2, 6-dimethyl-4-nitro-benzyl) - 2 - (trifluoromethyl) phenol

2-(4-methoxy-3-(trifluoromethyl)benzyl)-1,3-dimethyl-5-nitrobenzene (1.41g, 4.15mmol) and pyridine hydrochloride (17.0g) were stirred at 160°C for 16h. After the reaction was completed, the temperature was lowered to room temperature. Ethyl acetate (200mL) and water (200mL) were added into the solution, and the reaction was stirred for 30 min. The liquid was partitioned, and the aqueous phase was extracted with ethyl acetate (100mL). The organic phases were combined and washed with saturated aqueous sodium chloride (200 mL). The combined organic phase was dried with anhydrous magnesium sulfate, then filtered. The filtrate was concentrated and purified by reversed-phase preparative liquid phase purification ( mobile phase A: water, mobile phase B: acetonitrile, gradient: 5-75% (%B)) to give 945 mg of product in 70% yield.MS (ESI) m/z: 326.3 [M + H]⁺_{∘}

### step 4 4 - (4-amino-2, 6-dimethyl-benzyl) - 2 - (trifluoromethyl) phenol

To a solution of 4-(2,6-dimethyl-4-nitrobenzyl)-2-(trifluoromethyl)phenol (2.73 g, 8.38 mmol) in tetrahydrofuran (20 mL) and anhydrous methanol (20 mL), ammonium chloride aqueous solution(4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.30 g of product in 93% yield. MS (ESI) m/z: 296.3 [M + H]⁺_{∘}

### Step 5 (2-cyano-2 - (2 - (4 - (4-hydroxy-3 - (trifluoromethyl) benzyl) -3, 5-dimethyl-phenyl) - hydrazino) acetyl) carbamic acid ethyl ester

To a solution of 6M aqueous hydrochloric acid (40 mL) in anhydrous ethanol (30 mL), 4-(4-amino-2,6-dimethylbenzyl)-2-(trifluoromethyl)phenol (2.29 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture was continued to stir for 30 min at 0-5°C. A solution of pyridine (30 mL), water (40 mL) of n-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.68 g of product in 75% yield.MS (ESI) m/z: 463.4 [M + H]⁺_{∘}

### step 6 2 - (4 - (4-hydroxy-3 - (trifluoromethyl) benzyl) -3, 5-dimethyl-phenyl) - 3, 5-dioxo-2, 3, 4, 5-tetrahydro-1, 2, 4-triazine-6-carbonitrile

To a solution of (2-cyano-2-(2-(2-(4-(4-hydroxy-3-(trifluoromethyl)benzyl)-3,5-dimethylphenyl)hydrazinylidene)acetyl)carbamic acid ethyl ester (2.62 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.18 g of product in 50% yield. ¹H NMR (400 MHz, DMSO-d6): δ 12.95 (br s, 1H), 9.50 (s, 1H), 7.42(s, 2H), 7.32 (d, 1H), 7.08 (d, 1H), 6.76(d, 1H), 3.96(s, 2H), 2.18 (s, 6H).

### Example 10 2 - (4 - (4-hydroxy-3 - (deuterated methyl) benzyl) -3, 5-dimethylphenyl) -3, 5-dioxo-2, 3, 4, 5-tetrahdro-1, 2, 4-triazin-6-carbonitrile (Compound 10)

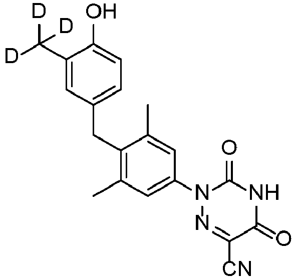

### step 1 (2, 6-dimethyl-4-nitrophenyl)-(4-methoxy-3 - (deuterated methyl) phenyl) methanol

To a solution of 2-bromo-1,3-dimethyl-5-nitrobenzene (2.83 g, 12.3 mmol) in tetrahydrofuran (80 mL) was added n-butyllithium (10 mL, 24.5 mmol) dropwise at -70° C.
After stirred at -70° C for 20 min, 4-methoxy-3-(deuteromethyl)benzaldehyde (2.25 g, 14.7 mmol) in tetrahydrofuran (20mL) was added dropwise into the above reaction solution, and the reaction was stirred at - 70° C for 60min. After the reaction was completed, the reaction solution was poured into saturated aqueous ammonium chloride solution (300mL). The mixture was extracted with ethyl acetate (150mL × 2) and the organic phases were combined. The organic phase was washed with saturated sodium chloride (200 mL), then dried over anhydrous magnesium sulfate and filtered. The filtrate was concentrated and purified by reversed-phase preparative liquid phase purification (mobile phase A: water, mobile phase B: acetonitrile, gradient: 10-95% (%B)) to give 2.55 g of product in 68% yield.MS (ESI) m/z: 305.4 [M + H]⁺_{∘}

### step 2 2 - (4-methoxy-3 - (deuterated methyl) benzyl) -1, 3-dimethyl-5-nitrobenzene

To a solution of (2,6-dimethyl-4-nitrophenyl)(4-methoxy-3-(deuteromethyl)phenyl)methanol (2.36 g, 7.74 mmol) in dichloromethane (40 mL), trifluoroacetic acid (10 mL) and triethylsilane (20 mL) were added dropwise at room temperature. The reaction was stirred at room temperature overnight. After the reaction was completed, the reaction solution was poured into saturated aqueous sodium bicarbonate solution and extracted by dichloromethane (300 mL × 2).The organic phases were combined and washed with saturated aqueous sodium chloride solution (250 mL). The combined organic phase was dried with anhydrous magnesium sulfate, then filtered. The filtrate was concentrated and purified by reversed-phase preparative liquid phase purification (mobile phase A: water, mobile phase B: acetonitrile, gradient: 5-85% (%B)) to give 1.45 g of product in 65% yield.MS (ESI) m/z: 289.4 [M + H]⁺_{∘}

### step 3 4 - (2, 6-dimethyl-4-nitro-benzyl) - 2 - (deuterated methyl) phenol

2-(4-methoxy-3-(deuteromethyl)benzyl)-1,3-dimethyl-5-nitrobenzene (1.20 g, 4.15 mmol) and pyridine hydrochloride (17.0g) were stirred at 160°C for 16h. After the reaction was completed, the temperature was lowered to room temperature. Ethyl acetate (200mL) and water (200mL) were added into the solution, and the reaction was stirred for 30min. The liquid was partitioned, and the aqueous phase was extracted with ethyl acetate (100mL). The organic phases were combined and washed with saturated aqueous sodium chloride (200 mL). The combined organic phase was dried with anhydrous magnesium sulfate, then filtered. The filtrate was concentrated and purified by reversed-phase preparative liquid phase purification ( mobile phase A: water, mobile phase B: acetonitrile, gradient: 5-75% (%B)) to give 910 mg of product in 80% yield.MS (ESI) m/z: 275.3 [M + H]⁺_{∘}

### step 4 4 - (4-amino-2, 6-dimethyl-benzyl) - 2 - (deuterated methyl) phenol

To a solution of 4-(2,6-dimethyl-4-nitrobenzyl)-2-(deuteromethyl)phenol (2.3 g, 8.38 mmol) in tetrahydrofuran (20 mL) and anhydrous methanol (20 mL), ammonium chloride aqueous solution (4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 1.95 g of product in 95% yield.MS (ESI) m/z: 245.3 [M + H]⁺_{∘}

### Step 5 (2-cyano-2 - (2 - (4 - (4-hydroxy-3 - (deuterated methyl) benzyl) -3, 5-dimethyl-phenyl) - hydrazino) acetyl) carbamic acid ethyl ester

To a solution of 6M aqueous hydrochloric acid (40 mL) in anhydrous ethanol (30 mL), 4-(4-amino-2,6-dimethylbenzyl)-2-(deuteromethyl)phenol (1.90 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture was continued to stir for 30 min at 0-5°C. A solution of pyridine (30 mL), water (40 mL) and n-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.42 g of product in 76% yield. MS (ESI) m/z: 412.5 [M + H]⁺_{∘}

### step 6 2 - (4 - (4-hydroxy-3 - (deuterated methyl) benzyl) -3, 5-dimethyl-phenyl) - 3, 5-dioxo-2, 3, 4, 5-tetrahydro-1, 2, 4-triazine-6-carbonitrile

To a solution of (2-cyano-2-(2-(2-(4-(4-hydroxy-3-(deuteromethyl)benzyl)-3,5-dimethylphenyl)hydrazinylidene)acetyl)carbamic acid ethyl ester (2.33 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%~80%) to give 1.1 g of product in 53% yield. MS (ESI) m/z: 366.4 [M + H]⁺_{∘} ¹H NMR (400 MHz, DMSO-d6): δ 12.87 (brs, 1H), 9.26 (s, 1H), 7.42(s, 2H), 6.93 (d, 1H), 6.87 (d, 1H), 6.71(d, 1H), 3.96(s, 2H), 2.10 (s, 6H).

### Example 11: 2 - (3, 5-dichloro-4 - (4-hydroxy-3 - (allyl) phenoxy) phenyl) -3, 5-dioxo-2, 3, 4, 5-tetrahydro-1, 2, 4-triazine-6-carbonitrile (compound 11)

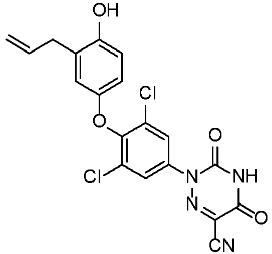

### Step 1: 4 - (2, 6-dichloro-4-nitrophenoxy) - 2 - (allyl) - phenol

To a solution of 2-allylbenzene-1,4-diol (3.00 g, 20.0 mmol) in acetonitrile (50 mL), sodium carbonate (7.57 g, 71.4 mmol) and 1,3-dichloro-2-fluoro-5 nitrobenzene (3.00 g, 14.3 mmol) were added. The reaction mixture was stirred at 48°C for 8 hours. Water (50 mL) and ethyl acetate (50 mL) were added to the concentrate, and the pH was adjusted to 2-3 with 10% aqueous HCl. The mixture was extracted with ethyl acetate (30 mL × 3) and the organic phases were combined. The organic phase was washed with saturated aqueous sodium chloride solution (10 mL), then dried with anhydrous sodium sulfate and filtered. The filtrate was concentrated and purified by silica gel column chromatography (eluent: petroleum : ethyl acetate = 20 : 1-15 : 1) to obtain 2.85 g of the target product in 59 % yield. [M+H]⁺: 340.0_{∘}

### Step 2: 4 - (4-amino-2, 6-dichloro-phenoxy) - 2 - (allyl) - phenol

To a solution of 4-(2,6-dichloro-4-nitrophenoxy)-2-(allyl)phenol (2.85 g, 8.38 mmol) in tetrahydrofuran (20 mL) and anhydrous methanol (20 mL), ammonium chloride aqueous solution(4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.42 g of product in 93% yield. [M+H]⁺: 310.1 .

### Step 3: (2-cyano-2 - (2 - (3, 5-dichloro-4 - (4-hydroxy-3-allyl-phenoxy) phenyl) - hydrazino) acetyl) carbamic acid ethyl ester

To a solution of 6M aqueous hydrochloric acid (40 mL) in anhydrous ethanol (30 mL), 4-(4-amino-2,6-dichlorophenoxy)-2-(allyl)phenol (2.40 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture continued to be stirred for 30 min at 0-5°C. A solution of pyridine (30 mL), water (40 mL) and n-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.7 g of product in 73% yield. [M+H]⁺: 477.1_{∘}

### Step 4: 2 - (3, 5-dichloro-4 - (4-hydroxy-3 - (allyl) phenoxy) phenyl) -3, 5-dioxo-2, 3, 4, 5-tetrahydro-1, 2, 4-triazine-6-carbonitrile

To a solution of (2-cyano-2-(2-(3,5-dichloro-4-(4-hydroxy-3-allylphenoxy)phenyl)hydrazone)acetyl)carbamic acid ethyl ester (2.70 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%-80%) to give 1.05 g of product in 43% yield.MS (ESI) m/z: 431.0 [M - H]⁻

### Example 12 2 - (4 - (4-hydroxy-3 - (allyl) benzyl) -3, 5-dimethylphenyl) -3, 5-dioxo-2, 3, 4, 5-tetrahydro-1, 2, 4-triazin-6-carbonitrile (compound 12)

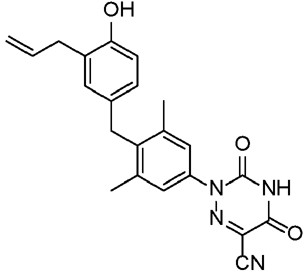

### step 1 (2, 6-dimethyl-4-nitrophenyl) (4-allyloxy-phenyl) methanol

To a solution of 2-bromo-1,3-dimethyl-5-nitrobenzene (2.83 g, 12.3 mmol) in tetrahydrofuran (80 mL), n-butyllithium (10 mL, 24.5 mmol) was added dropwise at -70° C. After being stirred at -70° C for 20 min, 4-allyloxy-benzaldehyde (3.0 g, 14.7 mmol) in tetrahydrofuran (20mL) was added dropwise into the above reaction solution, and the reaction was stirred at -70° C for 60min. After the reaction was completed, the reaction solution was poured into saturated aqueous ammonium chloride solution (300mL). The mixture was extracted with ethyl acetate (150mL × 2) and the organic phases were combined. The organic phase was washed with saturated sodium chloride (200 mL), then dried over anhydrous magnesium sulfate and filtered. The filtrate was concentrated and purified by reversed-phase preparative liquid phase purification (mobile phase A: water, mobile phase B: acetonitrile, gradient: 10-95% (%B)) to give 2.84 g of product in 65% yield.MS (ESI) m/z: 314.1 [M + H]⁺_{∘}

### step 2 2 - (4-allyloxy) - benzyl -1, 3-dimethyl-5-nitrobenzene

To a solution of (2,6-dimethyl-4-nitrophenyl)(4-allyloxyphenyl) methanol (2.75 g, 7.74 mmol) in dichloromethane (40 mL), trifluoroacetic acid (10 mL) and triethylsilane (20 mL) were added dropwise at room temperature. The reaction was stirred at room temperature overnight. After the reaction was completed, the reaction solution was poured into saturated aqueous sodium bicarbonate solution and extracted by dichloromethane (300 mL × 2).The organic phases were combined and washed with saturated aqueous sodium chloride solution (250 mL). The combined organic phase was dried with anhydrous magnesium sulfate, then filtered. The filtrate was concentrated and purified by reversed-phase preparative liquid phase purification (mobile phase A: water, mobile phase B: acetonitrile, gradient: 5-85% (%B)) to give 1.58 g of product in 60% yield. MS (ESI) m/z: 298.2 [M + H]⁺_{∘}

### step 3 4 - (2, 6-dimethyl-4-nitro-benzyl) - 2 - (allyl) - phenol

To a solution of 2-(4-allyloxy)benzyl-1,3-dimethyl-5-nitrobenzene (1.41 g, 4.15 mmol) in dichloromethane (20 mL) was added diethylaluminium chloride (1.2 eq). The reaction mixture was stirred at room temperature for 3 h. The mixture was quenched by adding water (5 mL), and stirred for 30 min. The liquid was partitioned, and the aqueous phase was extracted by dichloromethane (100 mL). The organic phases were combined and washed by saturated aqueous sodium chloride solution (200 mL). The combined organic phase was dried with anhydrous magnesium sulfate, then filtered. The filtrate was concentrated and purified by reversed-phase preparative liquid phase purification (mobile phase A: water, mobile phase B: acetonitrile, gradient: 5-75% (%B)) to give 945 mg of product in 70% yield. MS (ESI) m/z: 298.2 [M + H]⁺_{∘}

### step 4 4 - (4-amino-2, 6-dimethyl-benzyl) - 2 - (allyl) - phenol

To a solution of 4-(2,6-dimethyl-4-nitrobenzyl)-2-(trifluoromethyl)phenol (2.73 g, 8.38 mmol) in tetrahydrofuran (20 mL) and anhydrous methanol (20 mL), ammonium chloride aqueous solution(4.48 g, 83.8 mmol, 20 mL) and iron powder (2.82 g, 50.3 mmol) were added. The reaction was stirred at 70°C for 3 hours. After the reaction was complete, the reaction solution was cooled down to room temperature. Water (50 mL), saturated aqueous sodium bicarbonate solution (30 mL), and ethyl acetate (50 mL) were added to the reaction solution, stirred for 10 minutes, and then filtered. The filtrate was extracted with ethyl acetate (20 mL × 3) and the organic phases were combined. The organic phase was washed with saturated sodium chloride (50 mL), then dried over anhydrous magnesium sulfate and filtered. The filtrate was concentrated to dryness under reduced pressure to give 2.30 g of product in 93% yield. MS (ESI) m/z: 268.2 [M + H]⁺_{∘}

### Step 5 (2-cyano-2 - (2 - (4 - (4-hydroxy-3 - (allyl) benzyl) -3, 5-dimethyl-phenyl) - hydrazono) acetyl) carbamic acid ethyl ester

To a solution of 6M aqueous hydrochloric acid (40 mL) in anhydrous ethanol (30 mL), 4-(4-amino-2,6-dimethylbenzyl)-2-(allyl)phenol (2.29 g, 7.74 mmol) was added. The mixture was cooled down to 5°C and an aqueous solution of sodium nitrite (534 mg, 7.74 mmol) (3 mL) was added dropwise to the mixture. After dropwise addition, the mixture continued to be stirred for 30 min at 0-5°C. A solution of pyridine (30 mL), water (40 mL) andn-cyanoacetylurethane (1.12 g, 7.74 mmol) was added dropwise to the reaction solution, and the mixture was stirred at 5°C for 2 h. After the reaction was complete, water (30 mL) was added to the reaction solution. During filtering, the filter cake was drenched with water (10 mL × 3). The filter cake was dried under reduced pressure to give 2.68 g of product in 75% yield. MS (ESI) m/z: 435.2 [M + H]⁺_{∘}

### step 6 2 - (4 - (4-hydroxy-3 - (allyl) benzyl) -3, 5-dimethyl-phenyl) - 3, 5-dioxo-2, 3, 4, 5-tetrahydro-1, 2, 4-triazine-6-carbonitrile

To a solution of (2-cyano-2-(2-(2-(4-(4-hydroxy-3-(allyl)benzyl)-3,5-dimethylphenyl)hydrazinylidene)acetyl)carbamic acid ethyl ester (2.62 g, 5.66 mmol) in glacial acetic acid (30 mL), sodium acetate (928 mg, 11.3 mmol) was added. The mixture was heated to 118°C and stirred at 118°C for 3 hours. The reaction solution was cooled down to 60°C and concentrated to dryness under reduced pressure. The concentrate was purified by preparative liquid phase purification over a C18 column (mobile phase: acetonitrile: 0.1% aqueous ammonium bicarbonate = 20%-80%) to give 1.18 g of product in 50% yield.MS (ESI) m/z: 389.2 [M + H]⁺_{∘}

### Effect embodiment

**Effect embodiment 1:** TR-FRET (time-resolved fluorescence resonance energy transfer) thyroid receptor co-activation test

### Experimental Procedures

(1) The complete TR-FRET CotBuffer C was prepared by the addition of 1MDTT (dithiothreitol) to TR-FRET CofferBuffer C (purchased in Thermofisher), and the final concentration is 5mM DTT.
(2) 100nL of the compound to be tested with the concentration of 200X is added to each hole. If it is control group, then 100nL DMSO is added;
(3) adding 10 µL of complete TR-FRET Coffin Buffer C in each hole;
(4) preparing the TR-LBD (thyroid hormone ligand binding domain) of 4 X by using the pre-cooled Complete TR-fret Comm Buffer C;
(5) adding 5 µL of 4 X TR-LBD into the experiment plate; preparing complete TR-FRET Comm BufferC at room temperature with a solution containing 0.4 µM of the fluorescent enzyme-SRC2-2 (purchased from Thermofisher Corp.) (4 times) and 8 nM of Tbanti-GST (4-fold). 5 µL of 4 X peptide/4X antibody solution (purchased in Thermofisher Corporation) was added to the experimental plate.
(6) lightly mixing the 384-hole plate on the plate shaking machine, avoiding light incubation 2h at room temperature. using instrument set, the wavelength is 520nm and 495nm, and the specific parameter is as follows:

| | |
|---|---|
| Excitation Wavelength | 340 nm Bandpass Filter □ 30 nm bandwidth□ |
| Emission Wavelength | 520 nm Bandpass Filter □ 25 nm bandwidth□ |
| Emission Wavelength | 490 nm or 495 nm Bandpass Filter (10 nm bandwidth) |
| Lag Tim e | 100 µs |
| Integration Time | 200 µs |

**Test compounds:** Transthyretin T3 (positive control), MGL3196 (positive compound), the compound of the embodiment of the present invention.

### Experimental Results:

| | TRa, TR- FRET Coactivator Assay | | | | TRβ TR- FRET Coactivator Assay | | | |
|---|---|---|---|---|---|---|---|---|
| | EC₅₀ (nM) | | %MAX of T3 | | EC₅₀ (nM) | | %MAX of T3 | |
| T3 | | 0.12 | | 100.00 | | 0.16 | | 100.00 |
| MGI3196 | | 602 | | 32.24 | | 60.7 | | 48.46 |
| compound 1 | | 4.27 | | 75.04 | | 0.86 | | 90.73 |
| compound 2 | | 868 | | 14.88 | | 169 | | 27.30 |
| compound 3 | | 2.62 | | 89.73 | | 4.47 | | 90.63 |

The results showed that compounds 1 and 3 of the present invention showed agonistic activities on both TR α and TR β, which were already close to the positive control T3, and the agonistic effect on TR β had reached more than 90% of the agonistic effect of T3. Both compounds also showed significantly higher EC50 values and agonistic effects on TR α and TR β than compound MGL3196.

**Effect embodiment 2:** Fluorescence enzyme functional test based on HEK293/TRβ-luc cell
(1) Composite plates were prepared and 125 nL of HEK293/TR β-luc cells were added to each well of the assay plate. The cell density was determined by counting and the cell suspension was diluted with complete medium at a cell density of 4 × 10⁵ /mL.
(2) adding 25uL cell to each hole in the detection plate containing the compound to be tested, incubating for 24h at 37 degrees centigrade and 5% CO₂ environment;
(3) adding 25 µL of Steady-Glo (Promega) in each hole;
(4) centrifuging for 2 minutes at 2000RPM to eliminate bubbles;
(5) incubating at room temperature for 10 minutes, using an Envision (Envision 2105 model, PerkinElmer company) reading board.

**Test compounds:** Transthyretin T3 (positive control), MGL3196 (positive control), test example compound.

### Experimental Results:

| | HEK293/TRα cells EC50 (nM) | HEK293/1Rβ cells EC50 (nM) | Selectivity Ratio |
|---|---|---|---|
| T3 | 4.56 | 1.914 | 2.4 |
| MGL3196 | 26890 | 3356 | 8.0 |
| compound 1 | 1859 | 83.8 | 22.2 |
| compound 3 | 334 | 74.14 | 4.5 |

The results display that the compound 1 of the invention 1 display the agonist activity to TRα and TRβ, the EC50 value is significantly higher than the compound MGL3196, the selectivity for the TRβ cell is 22.2 times of the TR α cell, and also significantly higher than MGL3196.

### Effect embodiment 3: NASH animal model effect and safety test

C57BL/6J male mice, starting from 6 weeks old, are continuously fed with a CDAA-HFD diet (choline deficiency levorotatory amino acid high fat diet), and after 6 weeks (42 days), the moulding is successful. The model through VLDL (very low density lipoprotein) damages liver triglyceride secretion, mouse serum ALT and AST increases, and in 3 weeks there is fatty degeneration and inflammation, and hepatic fibrosis occurs at 5-6 weeks. The model is developed into liver cirrhosis in 24 weeks, and portal vein high pressure and liver failure. CDAA-HFD diet can induce liver steatosis and fibrosis in a short time, and has no obesity, hyperglycemia and hypertriglyceridemia, and so on, strong reference meaning for NASH disease research project. The invention uses the CDAA-HFD model to simulate the pathological process and physiological state of NASH, and the test compound for the early stage NASH treatment effect.

### Specific implementation schemes are as follows:

| 1. Grouping and dosing regimens: Group | animal number | Dosage (mg/kg, mpk) | Administration method |
|---|---|---|---|
| Normal control group (conventional feed) | 12 | PBS | p.o. q.d, 42days |
| Model group (CDAA-HFD) | 16 | PBS | p.o. q.d, 42days |
| Non-Nobel Group | 12 | 100 | p.o. q.d, 42days |
| MGL-3196 | 6 | 3.0 | p.o. q.d, 42days |
| compound 1 group | 6 | 3.0 | p.o. q.d, 42days |

| | | | |
|---|---|---|---|
| Note: p.o means oral (intragastric) administration; and q.d means once daily | | | |

The self-molding was successfully grouped for 42 days, and then administered 42 days in accordance with the dosing regimen.

Drug preparation: the sample powder was weighed for each administration group, then put in a 5 ml centrifugal tube, and a proper amount of 0.5 %MC was added, then uniformly mixing via vortex oscillation, and preparing into a solution with a corresponding concentration of the existing preparation.

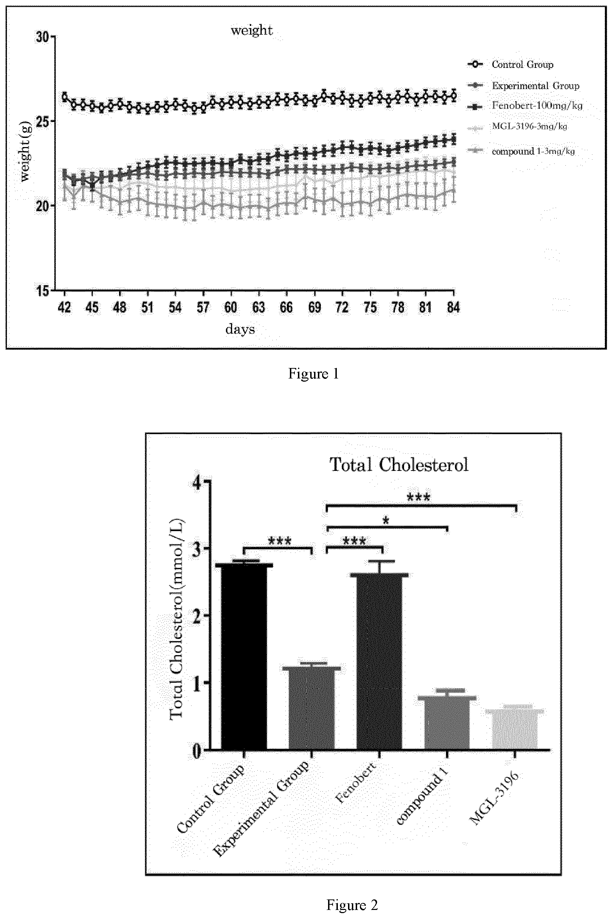

The state of the animal every time was observed and recorded. If the animal was killed, the animal was generally dissected, and the visceral abnormalities were observed by the naked eye, and recorded. During the experiment period, the weight of the animal was measured twice per week. The animal body weight curve of administration for 42 days is shown in FIG. 1

### 2. The Pathology Scoring

After weighing all mice at the corresponding time point, carbon dioxide was used with increased concentration to perform euthanason. Then cardiac puncture, taking blood, 7000 rpm for 10 min, taking plasma, immediately putting on dry ice, rotating and storing at -80 degrees centigrade. Subsequently the blood biochemical index was determined, comprising TCHO (total cholesterol), LDL (low density lipoprotein) and so on.

Also the liver was taken, and its weight was measured. Part of the liver was cut off (the same position of each animal) and fixed in 4 % of paraformaldehyde, for histopathological analysis (H-E staining and Tianwolf star red staining): liver fat and inflammatory cell infiltration degree, fibrosis and NAS score (NAS scoring system reference Chinese medical institute of non-alcoholic fatty liver disease diagnosis and treatment guide (2010 year revision), Ishak scoring system reference Journal of Hepatology 47 (2007) 598-607, Grading and III.).).

### 3. Statistical Analysis

The data is expressed by its average value + SEM. The statistical analysis of the difference between groups adopts single factor variance analysis (ANOVA), then using SPSS statistics software for a Dunnett test, where a P value less than 0.05 represents a statistical significance between two groups of data. In the scheme chart, * represents P < 0.05; ** represents P < 0.01; *** represents P < 0.001.

### 4. Results of Experiment

### 4.1 compound 1 of lipid-lowering effect

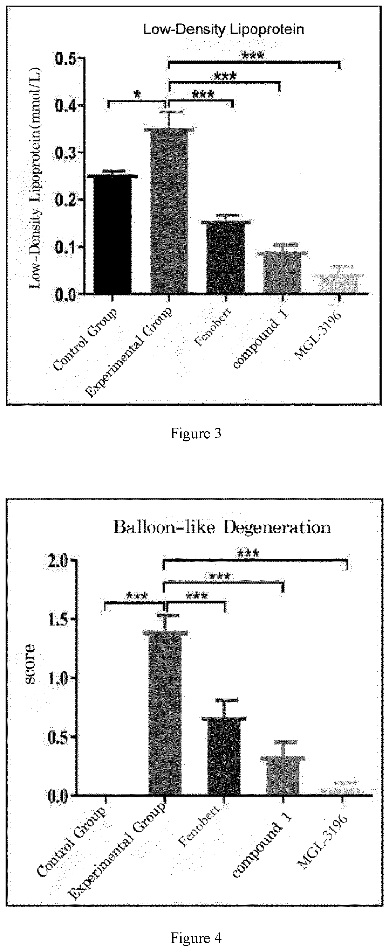

TCHO (total cholesterol) as shown in FIG. 2, LDL (low density lipoprotein) as shown in FIG. 3. Compound 1 comparing the normal diet group, the model group and the fenofibrate group has a significant decrease, which proves that the compound 1 as THR-β inhibitor can effectively reduce liver fat.

### 4. 2 compound 1 of hepatocyte gas ball sample change and inflammation score

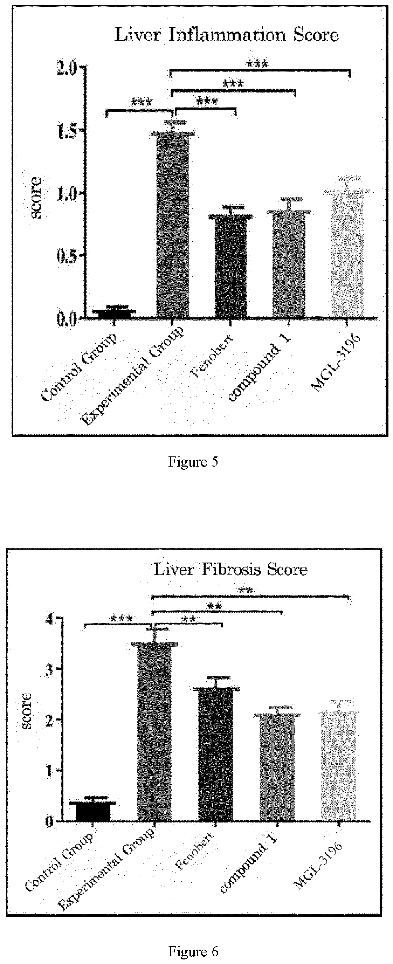

The change of the hepatocyte balloon can indicate the severity of the liver fat accumulation. As shown in FIG. 4, compound 1 can obviously reduce the hepatocyte gas ball sample variation value. The other main index of NASH is the liver inflammation cell infiltration degree. As shown in FIG. 5, compound 1 shows the good effect of inhibiting and reversing liver inflammation, showing that compound 1 has the potential for treating NASH.

### 4. 3 Compound 1 of fibrosis and NAS score

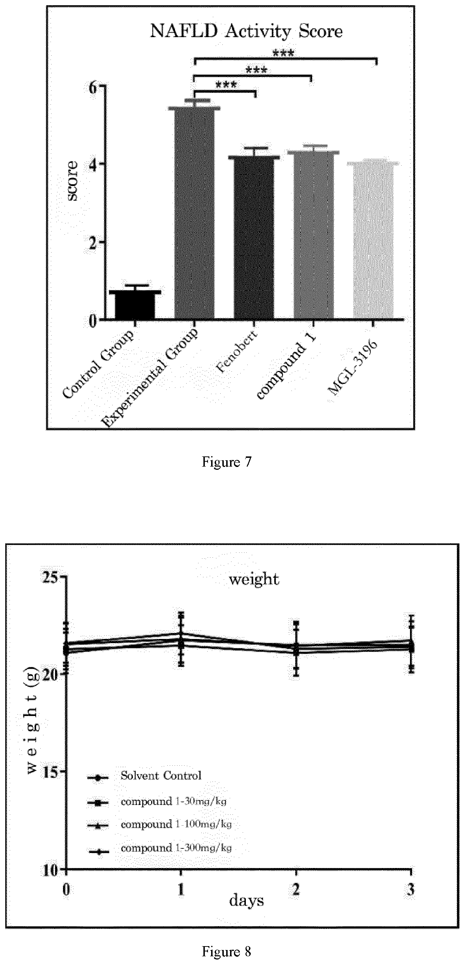

The liver fibrosis score as shown in FIG. 6, the NAS score as shown in FIG. 7, show that compound 1 significantly improves the liver NAS score and fibrosis degree.

The same method was used to test compound 2 for a lipid-lowering effect, by comparing compound 2 of the normal diet group, model group and fenofibrate group are significantly reduced, confirming that compound 2 as THR-beta inhibitor can effectively reduce liver fat. The same method was used to test compound 2 for the hepatocyte gas ball sample change and inflammation score. This shows that compound 2 also shows good effect of inhibiting and reversing liver inflammation.

### Effect Embodiment 4: The maximum tolerated dose test

C57BL/6J mice, 24, half male and half female, single admisnistration through mouth and stomach of 30, 100, 300mg/kg of compound 1, the solvent is 0.1% tween80 + 0.5 % MC water solution. After administration, clinical symptoms and weight was recorded, once a day, continuously for 3 days. On the fourth day, euthanasia was performed, and blood was collected for hematology, and blood biochemical detection.

The weight results are shown in FIG. 8. The mice have good tolerance to compound 1. The weight and clinical symptoms were not significantly abnormal during the experiment. Hematology, blood biochemical result were also not abnormal. The maximum tolerance (MTD) is more than 300mg/kg. The experiment result shows that compound 1 has good safety.

### Effect Embodiment 5: hERG test

Full automatic patch clamp QPatch technology was used, testing compound 1 to inhibit the CHO cell hERG potassium current.

### Specific implementation schemes are as follows:

### 1. Preparation of Cell

CHO-hERG cell culture is added in a 175cm² culture bottle, the cell density grew to 60 to 80 %, then the culture solution was removed, 7 mL PBS (Phosphate Buffered Saline phosphate buffer solution) was used for washing once, then 3 mL Detachin was added for digestion. After complete digestion, 7 mL culture liquid was added and then centrifuged, so the the supernatant was absorbed, then 5 mL culture liquid was added to re-suspend, to ensure the cell density was 2 ~ 5 × 10⁶/mL_{∘}

### 2. The electrophysiological recording process

Single cell high impedance sealing and the whole cell model process is automatically finished by the Qpatch instrument, the full cell model is obtained, and cell clamp is -80 millivolt, before a 5 seconds + 40 millivolt depolarization stimulation is carried out, firstly applying a 50 millisecond pre-voltage of -50 millivolts, then re-polarizing to -50 millivolt for 5 seconds, and then returning to -80 millivolt. The voltage stimulation is applied every 15 seconds, recorded for 2 minutes, the extracellular fluid is recorded for 5 minutes, then the administration process is started. The highest test concentration of compound 1 is 40.00 µ M, and sequentially it is 40.00, 13.33, 4.44, 1.48, 0.49, 0.16 µM. The DMSO content in the final test concentration is not more than 0.2 %. The concentration of DMSO does not affect the hERG potassium channel. Starting the compound concentration from the lowest test concentration, each test concentration is administered for 2.5 minutes, after continuously feeding all the concentrations, the positive control compound Cisapride is given. Each concentration at least tests 3 cells (n is more than or equal to 3).

### 3. Data Analysis

The experimental data is analyzed by GraphPad Prism 5.0 software.

### 4. Results of Experiment

| Compound | maximum test concentration (µM) | maximum concentration inhibition rate (%) | IC50□ µM□ |
|---|---|---|---|
| Cisapride | 3 | 98.9 | 0.023 |
| Compound 1 | 13.3 | 20.7 | □ 13.3 |

| | | | |
|---|---|---|---|
| Note: Compound 1 can not maintain normal sealing when the concentration of 40 µM, the data of this concentration is not included in the statistics. | | | |

The results demonstrate that compound 1 exhibits minimal impact on CHO cell hERG potassium currents, suggesting a lower likelihood of potential cardiac safety concerns.

## Claims

1. A compound represented by formula I or a pharmaceutically acceptable salt thereof, wherein the structure is as follows: 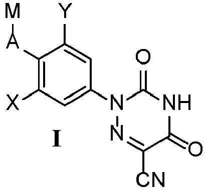
wherein A is O or CH₂;
M is; 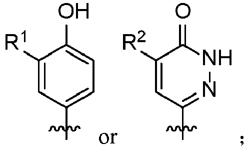
X and Y are independently chlorine, bromine, iodine, isotope ¹²⁴I or ¹³¹I of I or C₁ to C₆ alkyl;
R¹ is hydrogen, C₁ - C₆ alkyl, C₂ - C₆ alkenyl, one or more fluorine-substituted C₁ - C₆ alkyl, one or more fluorine-substituted C₂ - C₆ alkenyl, one or more deuterium-substituted C₁ - C₆ alkyl or one or more deuterium-substituted C₂ - C₆ alkenyl;
R² is C₂ - C₆ alkenyl, one or more fluorine-substituted C₁ - C₆ alkyl, one or more fluorine-substituted C₂ - C₆ alkenyl, one or more deuterium-substituted C₁ - C₆ alkyl or one or more deuterium-substituted C₂ - C₆ alkenyl.

2. The compound represented by formula I or a pharmaceutically acceptable salt thereof according to Claim 1, wherein X and Y are independently chlorine,bromine, iodine or CH₃;
and/or, R¹ is C₂ to C₆ alkenyl or " one or more fluorine substituted C₁ to C₆ alkyl ";
and/or, R² is one or more fluorine-substituted C₁ to C₆ alkyl.

3. The compound represented by formula I or a pharmaceutically acceptable salt thereof according to Claim 2, wherein when R¹ is C₂ to C₆ alkenyl, the C₂ to C₆ alkenyl is 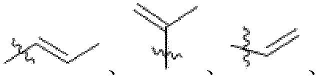 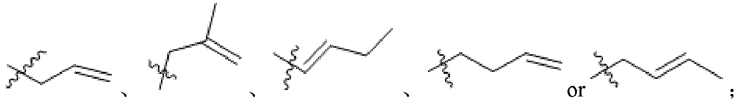 for example, or 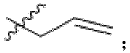
and/or, when R¹ is C₁ - C₆ alkyl, the C₁ - C₆ alkyl is methyl, ethyl, n-propyl, isopropyl, n-butyl, isobutyl, sec-butyl or tert-butyl, preferably isopropyl;
and/or, when R¹ is " one or more fluorine-substituted C₁ to C₆ alkyl ", the C₁ to C₆ alkyl is methyl, ethyl, n-propyl, isopropyl, n-butyl, isobutyl, sec-butyl or tert-butyl; one or more of the " one or more " is 1 or 3, preferably is 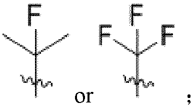
and/or, when R¹ is " one or more deuterium substituted C₁ to C₆ alkyl ", the C₁ to C₆ alkyl is methyl, ethyl, n-propyl, isopropyl, n-butyl, isobutyl, sec-butyl or tert-butyl; one or more of the " one or more " is 1 or 3, preferably is 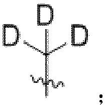
and/or, when R¹ is " one or more fluorine substituted C₂ to C₆ alkenyl ", the C₂ to C₆ alkenyl is 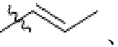 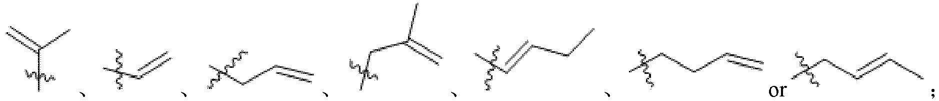
and/or, when R¹ is " one or more deuterium substituted C₂ to C₆ alkenyl ", the C₂ to C₆ alkenyl is 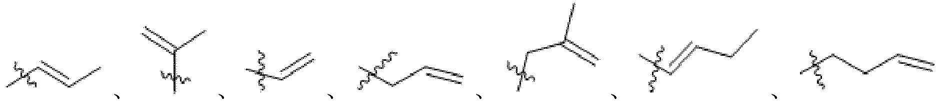 or 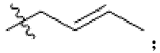 said " one or more " is 1 or 3;
and/or, when R² is C₂ to C₆ alkenyl, the C₂ to C₆ alkenyl is 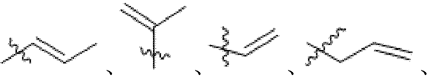 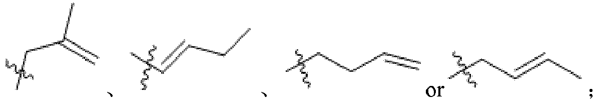 for example, 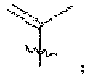
and/or, when R² is one or more fluorine-substituted C₁ - C₆ alkyl, the C₁ - C₆ alkyl is methyl, ethyl, n-propyl, isopropyl, n-butyl, isobutyl, sec-butyl or tert-butyl; one or more of the " one or more " is 1 or 3, preferably is 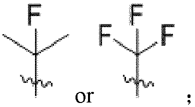
and/or, when R² is one or more deuterium-substituted C₁ - C₆ alkyl, the C₁ - C₆ alkyl is methyl, ethyl, n-propyl, isopropyl, n-butyl, isobutyl, sec-butyl or tert-butyl; one or more of the " one or more " is 1 or 3, preferably is 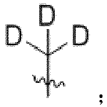
and/or, when R² is one or more fluorine-substituted C₂ to C₆ alkenyl, the C₂ to C₆ alkenyl is 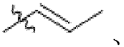 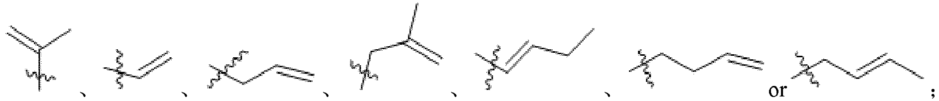 said " one or more " is 1 or 3;
and/or, when R² is one or more deuterium substituted C₂ to C₆ alkenyl, the C₂ to C₆ alkenyl is 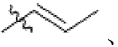 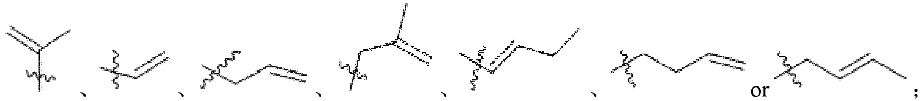 said " one or more " is 1 or 3;
and/or, when X is C₁ - C₆ alkyl, the C₁ - C₆ alkyl is methyl, ethyl, n-propyl, isopropyl, n-butyl, isobutyl, sec-butyl or tert-butyl; preferably methyl;
and/or, when Y is C₁ - C₆ alkyl, the C₁ - C₆ alkyl is methyl, ethyl, n-propyl, isopropyl, n-butyl, isobutyl, sec-butyl or tert-butyl; preferably methyl.

4. The compound represented by formula I or a pharmaceutically acceptable salt thereof according to any one of Claims 1-3, wherein it is defined as any one of the following schemes:
Scheme 1,
wherein A is O or CH₂;
X and Y are independently chlorine, bromine, iodine or C₁ to C₆ alkyl;
M is 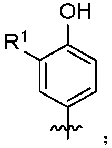 R¹ is hydrogen, C₁ - C₆ alkyl, C₂ - C₆ alkenyl, one or more fluorine-substituted C₁ - C₆ alkyl, one or more fluorine-substituted C₂ - C₆ alkenyl, one or more deuterium-substituted C₁ - C₆ alkyl or one or more deuterium-substituted C₂ - C₆ alkenyl,
or, M is 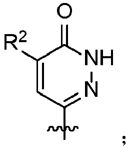 R² is one or more fluorine-substituted C₁ to C₆ alkyl;
Scheme 2
wherein A is O; X and Y are independently chlorine, bromine or iodine;M is 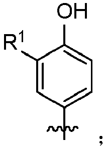 R¹ is C₂ to C₆ alkenyl or " one or more fluorine substituted C₁ to C₆ alkyl ";
Scheme 3
wherein A is O, X and Y are independently chlorine or bromine; M is 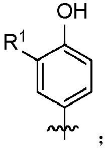 R¹ is C₂ - C₆ alkenyl or C₁ - C₆ alkyl substituted by one fluorine.

5. The compound represented by formula I or a pharmaceutically acceptable salt thereof according to Claim 4, wherein R¹ is 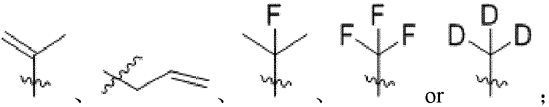 and/or, 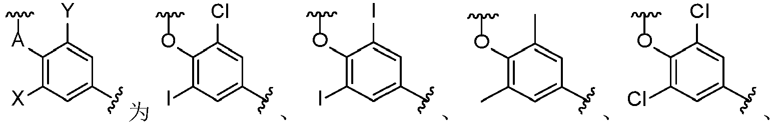 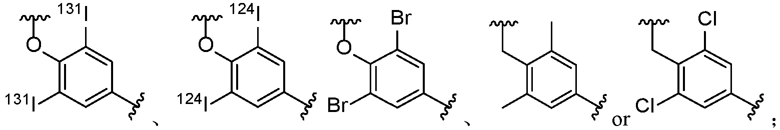 preferably, the compound represented by formula I is any one of the following compounds

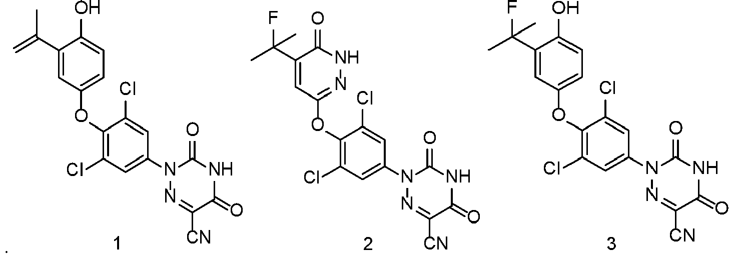

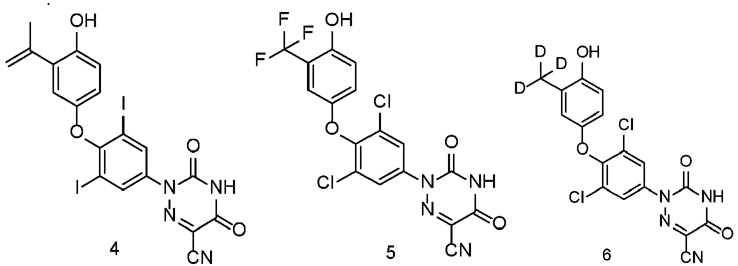

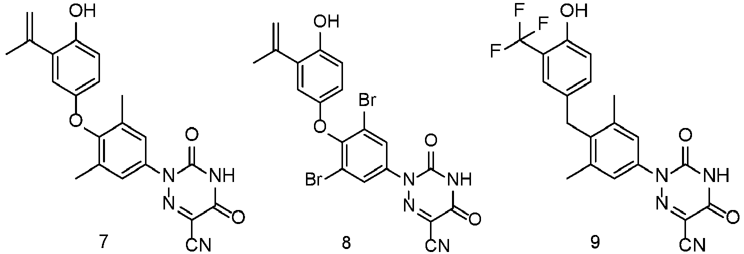

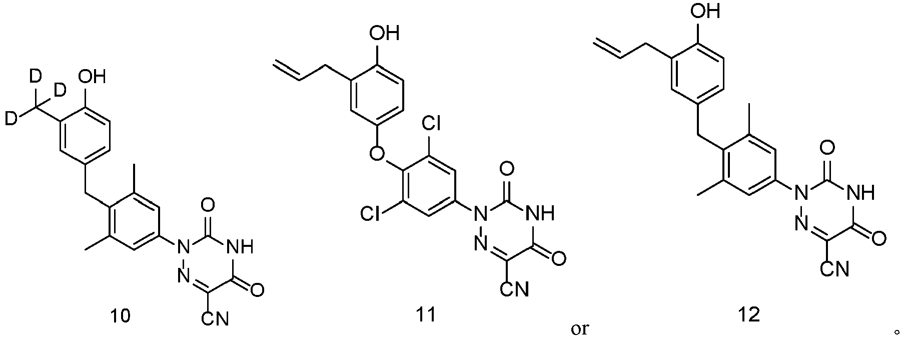

6. A preparation method of a compound represented by formula I, wherein the method comprises the following steps: in the solvent, and in the presence of alkali, the compound represented by formula II-a is subjected to the following ring-closing reaction, to obtain the compound represented by formula I; wherein the definition of M, X, Y and A is according to any one of Claims 1-5, and R⁶ is C₁-C₆ alkyl;

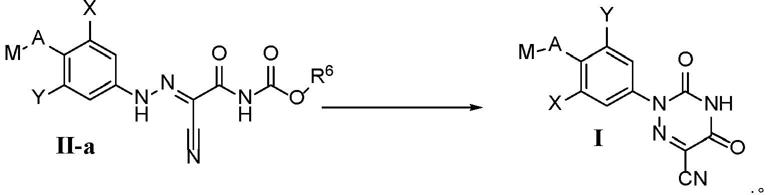

7. A compound represented by formula II-a, wherein, M, A, X and Y are defined according to any one of Claims 1 to 5, and R⁶ is defined according to Claim 6; 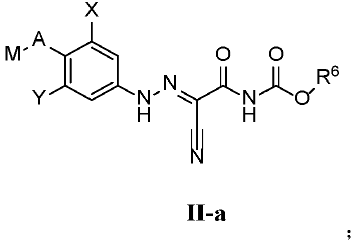 preferably, the compound represented by formula II-a is any one of the following compounds:

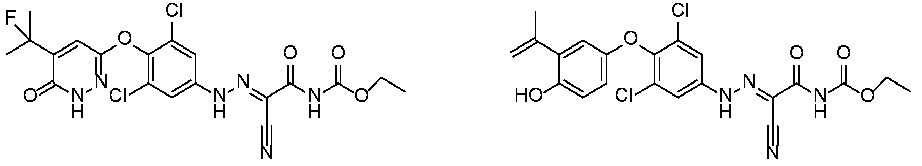

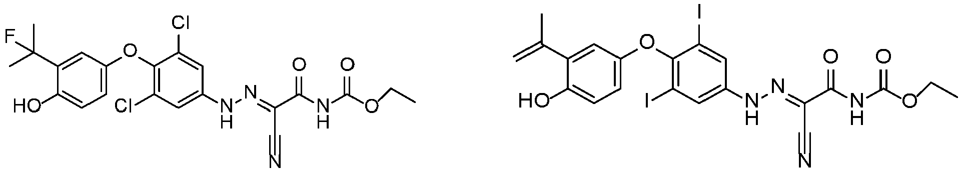

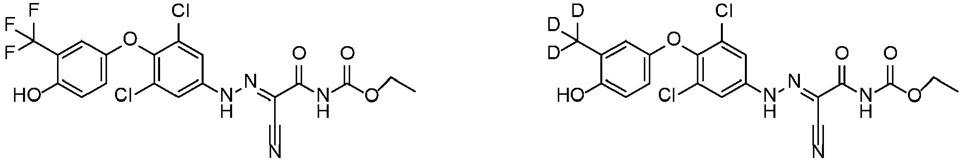

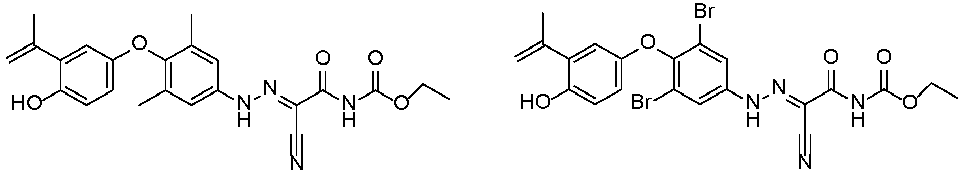

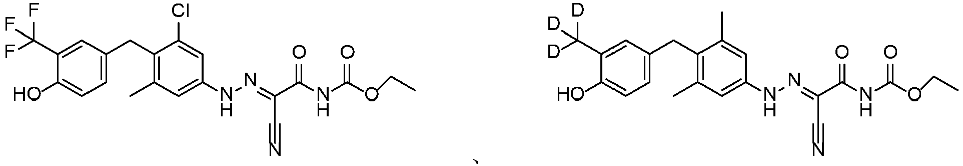

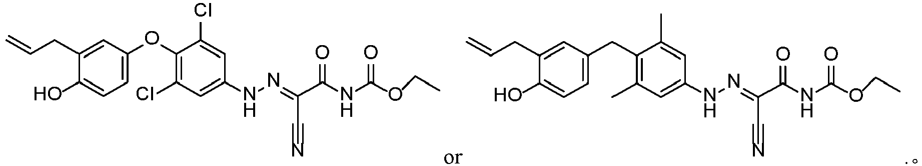

8. A pharmaceutical composition comprising substance A and one or more pharmaceutically acceptable carriers; the substance A is the compound represented by formula I or a pharmaceutically acceptable salt thereof according to any one of Claims 1-5.

9. An application of substance B in preparing a THR-β agonist agent wherein the substance B is the compound represented by formula I or a pharmaceutically acceptable salt thereof according to any one of Claims 1 to 5, or the pharmaceutical composition according to Claim 8.

10. Use of a substance B in the manufacture of a medicament for the treatment and/or prophylaxis of diseases associated with THR-β, wherein the substance B is the compound represented by formula I or a pharmaceutically acceptable salt thereof according to any one of Claims 1-5, or the pharmaceutical composition according to Claim 8; preferably, the disease is selected from one or more of non-alcoholic fatty liver disease, obesity, liver fibrosis, 2 type diabetes and primary hypercholesterolemia.
